# NEW EUROPEAN PATENT SPECIFICATION

(11) **EP 1 070 068 B2**
(45) Date of publication and mention of the opposition decision: **17.01.2007**
(45) Mention of the grant of the patent: 08.10.2003
(21) Application number: 99908706.7
(22) Date of filing: 11.03.1999
(51) Int. Cl.: C07D 487/14, A61K 31/4188

(54) **GRANULATIMIDE DERIVATIVES FOR USE IN CANCER TREATMENT**
GRANULATIMIDE-DERIVATE ZUR BEHANDLUNG VON KREBS
DERIVES DE GRANULATIMIDE UTILISES DANS LE TRAITEMENT DU CANCER

(30) Priority: 13.03.1998 CA 2232074; 14.08.1998 CA 2245029; 26.02.1999 US 258991
(43) Date of publication of application: 24.01.2001
(73) Proprietor: THE UNIVERSITY OF BRITISH COLUMBIA, Vancouver, British Columbia V6T 1Z3 (CA)
(72) Inventor: ANDERSEN, Raymond, J., Vancouver, British Columbia V6S 1Z6 (CA); ROBERGE, Michel, Vancouver, British Columbia V6R 2H4 (CA); SANGHERA, Jasbinder, Vancouver, British Columbia V5X 2H5 (CA); LEUNG, Danny, Coquitlam, British Columbia V3J 7C9 (CA); BERLINCK, Roberto G.S.,, CP780, CEP-13560-970 Sao Carlos, SP (BR); PIERS, Edward, Richmond, British Columbia V7C 4X7 (CA); BRITTON, Robert, Vancouver, British Columbia V6R 2W5 (CA)
(74) Representative: Brasnett, Adrian Hugh
(86) International application number: PCT/CA1999/000224
(87) International publication number: WO 1999/047522

(56) References cited:
- EP-A- 0 328 000
- EP-A- 0 434 057
- EP-A- 0 657 458
- EP-A- 0 672 668
- EP-A- 0 735 038
- EP-A- 0 841 337
- WO-A-91/09034
- WO-A-94/04541
- WO-A-94/07895
- WO-A-95/32974
- WO-A-95/32975
- WO-A-95/32976
- WO-A-96/11933
- WO-A-97/18809
- WO-A-97/19080
- DE-A- 3 835 842
- P.SMITH ET AL: "The Chemistry of 3-(alpha-Cyanobenzylidene)-1-phenyltriazen es and Their Conversion to Diarylmaleimides and Phenanthrene-9,10-dicarboxamides" J.ORG.CHEM., vol. 55, no. 10, 1990, pages 3351-3362, XP002106645
- Hodges et al (1996) J. American Chemical Society, 118(30), 7117-7127
- Artico et al (1989) J. Heterocyclic Chemistry 26(2), 503-7
- Wald et al (1980)Chemische Berichte, 113(9), 2884-90
- Terpin et al (1998) Tetrahedron 54, 1745-1752
- Statement from the British Library regarding date of public availability of D4
- Berlinck et al (1998)J. Organic Chemistry 63, 9850-9856
- Roberge et al (1998) Cancer Research 58, 5701-5706
- Product description sheet for staurosporine
- Product description sheet for rebeccamycin

## Description

### BACKGROUND

Natural products are a rich source of novel organic compounds, many of which have interesting and desirable biological activities. From extracts of an organism of interest, such as marine invertebrates, methods of chemical separation and analysis may be applied to elucidate the structure of biologically active compounds. These chemical structures then form the basis for synthetic modifications to enhance the desired activity.

Marine ascidians, or sea squirts, have a number of unique secondary metabolites with potent biological activity. Colonial ascidians within the family *Didemnidae* have been particularly prolific, containing nitrogenous amino acid derived metabolites, including various cyclic peptides. The compounds termed didemnimides were isolated from *Didemnum conchyliatum.* These compounds are indole-maleimide-imidazole alkaloids, which could hypothetically be synthesized as a condensation of tryptophan and histidine.

The further study of *Didemna* products, and the characterization of their active agents, is of particular interest for the development of novel therapeutic agents and uses thereof.

### Relevant Literature

Didemnimide compounds are described by Vervoot *et al*. (1997) J. Org. Chem. **62**:1486-1490. In describing synthesis of such didemnimide compounds, Terpin *et al.* (1998) Tetrahedron **54:**1740-1752 reported a ring closing event that may occur upon irradiation or upon recrystallization from methanol of a Boc protected intermediate used in the didemnimide synthesis scheme.

The formation of a variety of substituted diarylmaleimides is described by Smith et al. (1990) J. Org. Chem. 55:3351-3362

Known G2 checkpoint inhibitors include purine analogues, *e.g*. caffeine, pentoxifylline, 2-aminopurine, 6-dimethylaminopurine; and staurosporine with its derivative UCN-01 (7-hydroxystaurosporine). See, for example, Busse *et al.* (1978) Radiat. Res. **76**:292-307; Schlegel (1986) Science **232:**1264-1266; Downes *et al.* (1990) J. Cell. Biol. **110**:1855-1859; Steinmann *et al.* (1991) P.N.A.S. **88:**6843-6847; Andreasson *et al.* (1992) P.N.A.S. **89:**2272-2276; Tam *et al.* (1992) Cell Growth Differ. **3**:811-817; and Wang *et al.* 1996) J. Nat'l Cancer Inst. **88**:956-965.

Experiments employing cells deficient in the tumour suppressor protein p53 have demonstrated the value of the two groups of G2 checkpoint inhibitors described above, for selectively sensitizing cancer cells to agents that damage DNA. Pentoxifylline has been shown to enhance cisplatin induced killing of p53-MCF-7 cells 30-fold and radiation induced killing of p53- A549 human lung adenocarcinoma cells 5-fold. For example, see Russell *et al.* (1995) Cancer Res. **55:**1639-1642; Powell *et al.* (1995) Cancer Res. **55:**1643-1648; Russell *et al.* (1996) Int. J. Radiat. Oncol. Biol. Phys. **36:**1099-1106; Yao *et al.* (1996) Nature Med. **2:**1140-1143; and Bracey *et al* (1997) Clin. Cancer Res. **3:** 1371-1381.

The treatment of certain cancers using anthracene compounds and pharmaceutical derivatives thereof is described in WO 96/11933 and European Patent EP 0 841 337

### SUMMARY OF THE INVENTION

Granulatimide compounds of Formula I are provided, as defined herein. This invention includes the naturally occurring compounds, granulatimide and isogranulatimide, in purified or partially purified form, including extracts containing these compounds taken from naturally occurring sources, *e.g. Didemnum granulatum.* In one embodiment of the invention, formulations of the compounds in combination with a physiologically acceptable carrier are provided. The pharmaceutical formulations are useful as a cytotoxic agent; as a protein kinase inhibitor; and to inhibit the G2 checkpoint This invention also provides the use of compounds of Formula I, *e.g.* to sensitize cells to the effects of DNA damaging agents; and the use of such compounds in the formulation of agents, including medicaments.

### BRIEF DESCRIPTION OF THE DRAWINGS

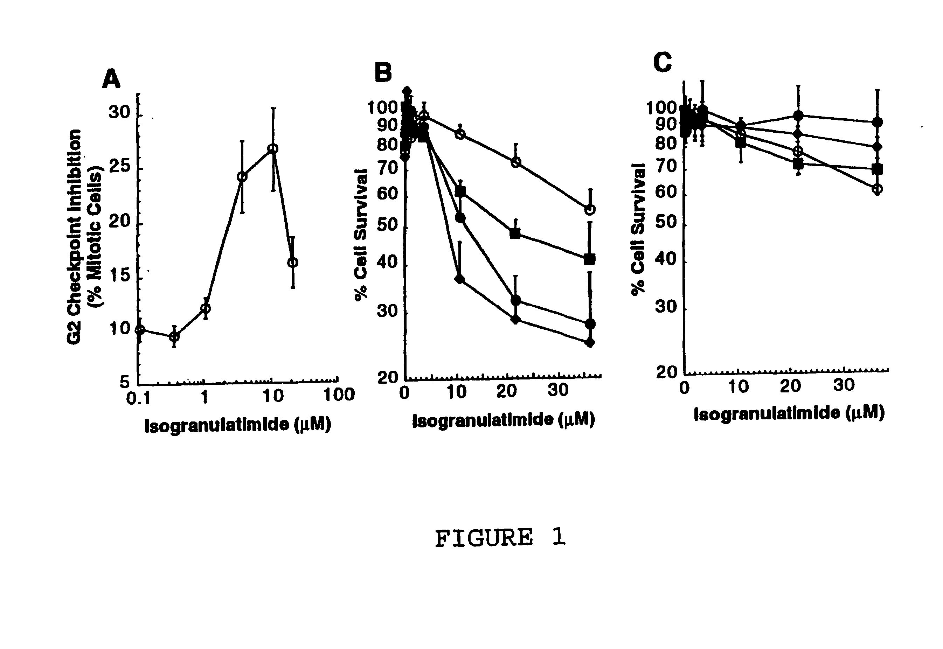

Figures 1A to 1C are a series of graphs showing: Fig. 1(A) G2 checkpoint inhibition by isogranulatimide; Fig. 1(B) isogranulatimide and γ-irradiation kill p53⁻ cells synergistically (MCF-7 mp53 cells seeded at 1000 cells per well in 96-well plates were grown overnight and irradiated with 0 Gy (○). 2 Gy (■), 4 (●) or 6 Gy (◆), immediately after irradiation the cells were treated with isogranulatimide for 16 h, cell survival was measured using a soluble tetrazolium salt assay (CellTiter96^{™}, Promega), 100% cell survival is defined as the surviving fraction after irradiation alone (6 Gy induced about 80% cell death); and Figure 1(C) iso-granulatimide and γ-irradiation do not kill p53+ cells synergistically (method identical to (B) except that MCF-7 cells are used).

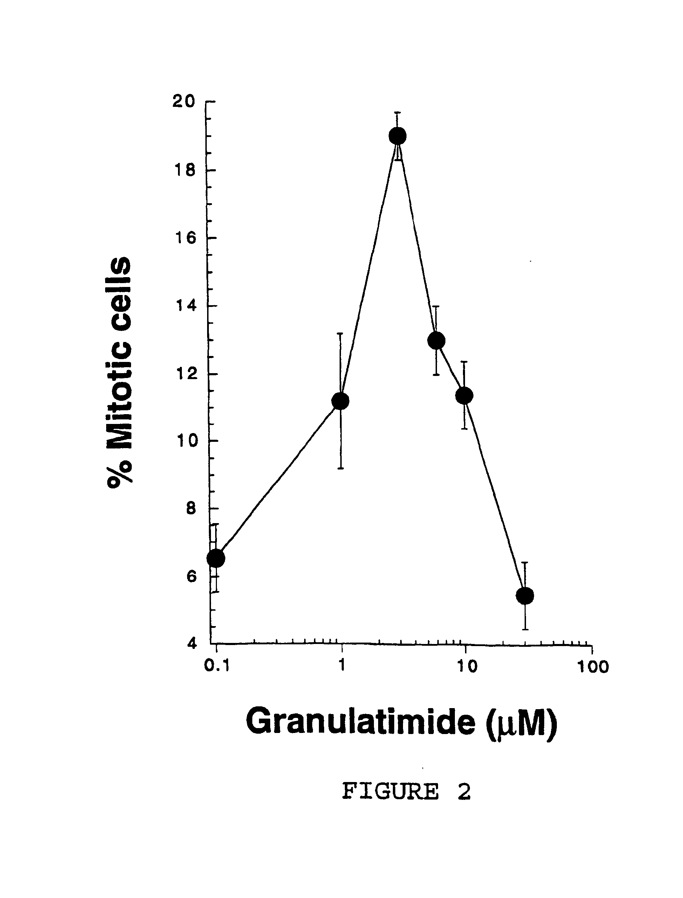

Figure 2 is a graph showing G2 checkpoint inhibition by granulatimide.

### DETAILED DESCRIPTION OF EMBODIMENTS OF THE INVENTION

Novel granulatimide compounds of Formula I are provided. This invention includes the naturally occurring compounds, granulatimide and iso-granulatimide, in purified or partially purified form, including extracts containing these compounds taken from naturally occurring sources. In one embodiment of the invention, formulations of the compounds in combination with a physiologically acceptable carrier are provided.

The pharmaceutical formulations are useful as a cytotoxic agent; and to inhibit protein kinases, including those that act to regulate the G2 checkpoint. Such G2 checkpoint inhibition prevents arrest, or releases cells that are arrested at this checkpoint, thereby permitting the cells to proceed to mitosis. This invention also provides the use of compounds of Formula I to sensitize cells to the effects of DNA damaging agents; and the use of such compounds in the formulation of agents, including medicaments, for potentiating the effect of DNA damaging agents on cells.

### DEFINITIONS

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention which will be limited only by the appended claims.

All technical and scientific terms used herein have the same meaning as commonly understood to one of ordinary skill in the art to which this invention belongs unless clearly indicated otherwise.

*Granulatimide compounds:* as used herein, the term "granulatimide compound" is used generically to describe a class of compounds having the structure as follows in Formula I. Included in this genus are the naturally occurring compounds granulatimide and isogranulatimide. Compounds of this invention designated herein as being compounds of Formula I have the structure given in claim 1, including all stereoisomers thereof when the compound may exist as stereoisomers:

Preferred compounds of Formula I are as follows, with substituents as defined above or as particularly defined below, where "Me" represents a methyl substituent throughout. 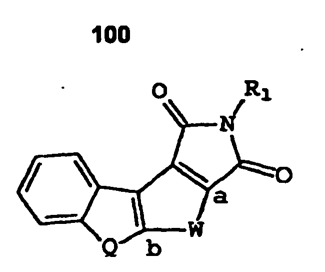 wherein Q=NH; R₁=H; and each of K, E, T = N or CH; 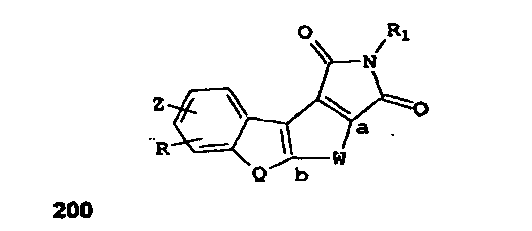 wherein Q=NH; R₁=H; and each of K, E, T = N or CH;

In one embodiment of the invention, the granulatimide compound is one of the naturally occurring compounds: granulatimide, or isogranulatimide.

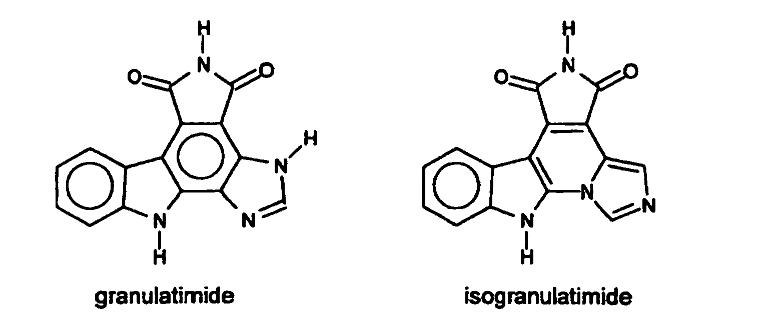

Pharmaceutically acceptable salts of the granulatimide compounds also fall within the scope of the compounds as disclosed herein. The term "pharmaceutically acceptable salts" as used herein means an inorganic acid addition salt such as hydrochloride, sulfate, and phosphate, or an organic acid addition salt such as acetate, maleate, fumarate, tartrate, and citrate. Examples of pharmaceutically acceptable metal salts are alkali metal salts such as sodium salt and potassium salt, alkaline earth metal salts such as magnesium salt and calcium salt, aluminum salt, and zinc salt. Examples of pharmaceutically acceptable ammonium salts are ammonium salt and tetramethylammonium salt. Examples of pharmaceutically acceptable organic amine addition salts are salts with morpholine and piperidine. Examples of pharmaceutically acceptable amino acid addition salts are salts with lysine, glycine, and phenylalanine.

*Protein Kinases and Inhibitors:* These enzymes use the gamma phosphate of ATP or GTP to generate phosphate monoesters utilizing protein alcohol groups on serine or threonine, and/or protein phenolic groups (tyrosine) as phosphate group acceptors. They are related by virtue of their homologous kinase domains, which consist of 200-300 amino acid residues. The kinase domains impart the catalytic activity by binding and orientation of the phosphate donor as a complex with divalent cation; binding and orientation of the polypeptide substrate; and transfer of the γ-phosphate from the ATP or GTP to the acceptor hydroxyl residue. Protein kinases are involved in signaling pathways for such important cellular activities as responses to extracellular signals and cell cycle checkpoints. Inhibition of specific protein kinases provides a means of intervening in these signaling pathways, for example to block the effect of an extracellular signal, to release a cell from cell cycle checkpoint, *etc*.

Defects in the activity of protein kinases are associated with a variety of pathological or clinical conditions, where there is a defect in signaling mediated by protein kinases. Such conditions include those associated with defects in cell cycle regulation or in response to extracellular signals, *e.g*. hyperglycemia and diabetes type I and type II, immunological disorders, *e.g*. autoimmune and immunodeficiency diseases; hyperproliferative disorders, which may include psoriasis, arthritis, inflammation, cancer, *etc*.

Protein kinases of interest include those involved in cell cycle regulation, particularly at the G2 checkpoint. Other kinases of interest include those involved in the GSK3 and ILK signaling pathways. Glycogen synthase kinase-3 (GSK3) is implicated in the regulation of several physiological processes, including the control of glycogen and protein synthesis by insulin, modulation of the transcription factors AP-1 and CREB, the specification of cell fate and dorsoventral patterning. GSK3 is inhibited by serine phosphorylation in response to insulin or growth factors and *in vitro* by either MAP kinase-activated protein (MAPKAP) kinase-1 or p70 ribosomal S6 kinase.

Integrin-linked kinase (ILK) is an ankyrin-repeat containing serine-threonine protein kinase capable of interacting with the cytoplasmic domains of integrin beta1, beta2, and beta3 subunits. Overexpression of ILK in epithelial cells disrupts cell-extracellular matrix as well as cell-cell interactions, suppresses suspension-induced apoptosis, and stimulates anchorage-independent cell cycle progression. In addition, ILK induces nuclear translocation of beta-catenin, where the latter associates with a T cell factor/lymphocyte enhancer-binding factor 1 (TCF/LEF-1) to form an activated transcription factor.

The granulatimide compounds of the present invention act as specific protein kinase inhibitors, and are useful as therapeutic agents in treating conditions characterized by defects in associated with these proteins. The compounds of the present invention are also useful in the manufacture of a medicament for inhibiting a protein kinase.

*G2 checkpoint.* Normal cells respond to DNA damage in one of two ways, depending upon their type and the degree of damage. The cells may activate an apoptotic pathway leading to suicide of the cell and its removal or, survival of a damaged cell may be promoted by activating checkpoints that temporarily halt the normal cycle of growth and division to allow time for DNA repair. The checkpoints operate during the G1 phase of the cycle so that DNA is repaired before it is replicated in the S phase; and, during the G2 phase so that DNA is repaired before chromosomes are segregated in the mitosis phase (M).

The subject granulatimide compounds are active in the inhibition of the G2 checkpoint. They are of interest as therapeutic reagents to treat cancer, etc. Administration of the subject compounds will release cells from the G2 checkpoint; and can result in apoptosis of the treated cells. Preferably the treated cells are unable to activate the G1 checkpoint. Many human tumors have mutations in the protein p53. As a result, they are unable to activate the G1 checkpoint, but can still utilize the G2 checkpoint. Inhibiting the checkpoint would drive tumor cells into mitosis, thereby increasing the effectiveness of cytotoxic agents that act by damaging DNA, *e.g*. cisplatin, bleomycin, and etoposide; and radiotherapy.

Over 50% of human cancers exhibit a loss of function of the protein p53. Cells with mutated p53 are unable to activate the G1 checkpoint in response to DNA damage. However, the G2 checkpoint (although usually weaker than in normal cells) still provides an opportunity to repair the DNA damage before cell division. Inhibition of the G2 checkpoint alone generally does not have a strong effect on normal cells, or tumor cells that retain normal G1 checkpoint activity. G2 checkpoint inhibitors used in combination with a DNA damaging agent will significantly increase the killing of cells that cannot activate the G1 checkpoint.

Throughout this specification, the term "G2" or G2 phase" means the phase of the cell cycle between the end of DNA synthesis and the beginning of mitosis. A cell in G2 has an interphase nucleus as determined by microscopy, and duplicated DNA (usually determined by flow cytometry).

*G2 checkpoint Inhibitor.* Throughout this specification, the term "G2 checkpoint inhibitor" means a substance which is capable of releasing a cell from arrest in G2 phase brought about by DNA damage, preventing a cell from arrest in G2 phase in response to DNA damage, or both.

This invention provides a method of increasing the killing by DNA damage of cells having G1 checkpoint deficiency, comprising the steps of administering a DNA damaging agent to said cells thereby damaging DNA of said cells, and administering a G2 checkpoint inhibitor compound to said cells, wherein the G2 inhibitor compound is a compound of Formula I, or a pharmaceutically acceptable salt thereof.

In a preferred embodiment, the present invention describes the use of a compound as described herein for the manufacture of a medicament for inhibiting the G2 checkpoint in a cell. Preferably the target cell is a hyperproliferative cell. The invention further encompasses the case when said hyperproliferative cell is p53 negative.

Throughout this specification, the term "DNA damaging agent" means any substance or treatment which induces DNA damage in a cell, including UV irradiation, gamma irradiation, X-rays, alkylating agents, antibiotics that induce DNA damage by binding to DNA, inhibitors of topoisomerases and any compound used in chemotherapy which acts by causing DNA damage. Examples of specific compounds are cisplatin, VM-26, and procarbazine.

*G2 Checkpoint Inhibitor Assay:* The G2 checkpoint inhibitor assay described herein involves treating cells, e. g. cells associated with a hyperproliferative disorder, including cancer cells, with a DNA damaging agent under conditions whereby arrest of the cells at G2 will be induced. A sample to be tested for G2 checkpoint inhibition activity and an agent which arrests cells in mitosis are applied to the cells, following which it is determined whether the G2 checkpoint is overcome and the cells enter mitosis. Release of the cells from arrest at G2 or prevention of arrest at G2, such that the cells proceed to mitosis, is the indicator of G2 checkpoint inhibition activity.

The assay requires use of a cell culture in which the cells are incapable of arrest at the G1 checkpoint in response to DNA damage. The cells may be from any of the numerous cell lines for which it is known that the cells are incapable of arrest at G1. Such cells include cells which are p53 deficient, including: any cell line with a natural mutation or deletion in the p53 gene which renders p53 inactive; any cell line expressing a viral oncogene which disrupts p53; any cell line expressing dominant-negative mutant p53 (some mutant p53 proteins inhibit wild-type protein such as in the p53- MCF-7 cells used in the examples herein); and any primary or established cell line derived from p53 "negative transgenic" animals. Specific examples of suitable cells are the following: cells which have incorporated the human papillomavirus type-16 E6 gene, cell lines which are mutant for p53 function including Burkitt's human lymphoma cell line CA46 and the human colon carcinoma cell line HT-29 (CA46 and HT-29 are available from the American Type Culture Collection). Cell lines with a genetic deficiency which disrupts the G1 checkpoint other than by disruption of p53 may also be employed in this assay.

Once a cell line incapable of G1 arrest is chosen, conditions for arresting a majority of the cells at G2 in response to a DNA damaging agent are optimized by determining appropriate culture conditions, incubation time, and type and dosage of DNA damaging agent. Preferably, at least 50% of the cells in a culture will be arrested at G2 in response to the DNA damaging agent. Maximizing the proportion of cells in the population which are arrested at G2 will reduce the background signal.

Once the conditions for G2 arrest in the cell culture are established, the assay may be carried out in one of two ways. First, the cells may be arrested at G2 in response to the DNA damaging agent and then treated with the sample to determine whether there is release from G2 arrest or, the cells may be treated with the sample prior to the time when the majority of the cells would be expected to be arrested at G2 and determine whether the cells are prevented from G2 arrest.

Release from G2 arrest or prevention of G2 arrest is detected by a quantitative determination of the cells which proceed to mitosis. The assay culture is treated with an agent which will arrest such cells in mitosis. Such agents include microtubule depolymerizing agents that arrest cells in metaphase, such as nocodazole. This will prevent cells from exiting mitosis and entering the next cell cycle.

In the assay, determination of the cells which proceed to mitosis is carried out using any of the known immunological methods by employing antibodies which have specificity for mitotic cells. Monoclonal antibodies demonstrating such specificity are known and include MPM-2 which was raised against mitotic HeLa cells and recognizes phospho-epitopes that are highly conserved in mitotic proteins of all eukaryotic species. Other examples are the monoclonal antibodies recognizing phospho-epitopes in the paired helical filament proteins (PHF) found in brain tissue of patients suffering from Alzheimer's disease as described in: PCT International Application published July 4, 1996 under No. WO 96/20218; and, Vincent, I. *et al.* (1996) "Mitotic Mechanisms in Alzheimer's Disease?" The Journal of Cell Biology, 132:413-425. The examples in this specification make use of the antibody TG-3 described in the latter two references, obtained from Albert Einstein College of Medicine of Yeshiva University, Bronx, New York.

TG-3 has a high specificity for mitotic cells. When TG-3 is used in the ELISA assay described herein, a very good signal/noise ratio is achieved permitting the ELISA assay to be easily monitored by optical absorbance.

Dying cells exhibit some characteristics which are similar to mitotic cells. TG-3 does not react with dying cells which prevents apoptotic cells from being counted in the assay of this invention. The efficiency of the assay is not affected by the presence of dying cells.

Immunological methods useful for determination of mitotic cells in this assay include any method for determining antibody-antigen binding, including: immunocytochemistry (eg. immunofluorescence), flow cytometry, immunoblotting, and ELISA. Several immunological methods are described in detail in the examples herein as well as in Vincent I. *et al.* [*supra*]. Other immunological procedures not described herein are well-known in the art and may be readily adapted for use in this assay. However, high throughput testing of samples may best be achieved by use of ELISA.

### METHODS OF SYNTHESIZING GRANULATIMIDE COMPOUNDS

This invention also provides processes for making compounds of Formula I. Various methods may be used, including syntheses as provided in the examples; isolation from natural sources; coupling of two compounds to produce a didemnimide followed by a cyclization step; *etc.* For example, one synthesis scheme starts with an appropriately protected indole compound such as indole-3-acetamide and proceeds over essentially two coupling steps to produce a didemnimide type compound, following by a cyclization step.The process may optionally comprise one or more subsequent steps to provide particular substituents required to produce a particular compound of Formula I.

An exemplary synthetic process is as follows, where P₁, P₂ and P₃ are suitable protecting groups. The starting indole compound (appropriately substituted by R and or Z) may be made by methods known in the art.

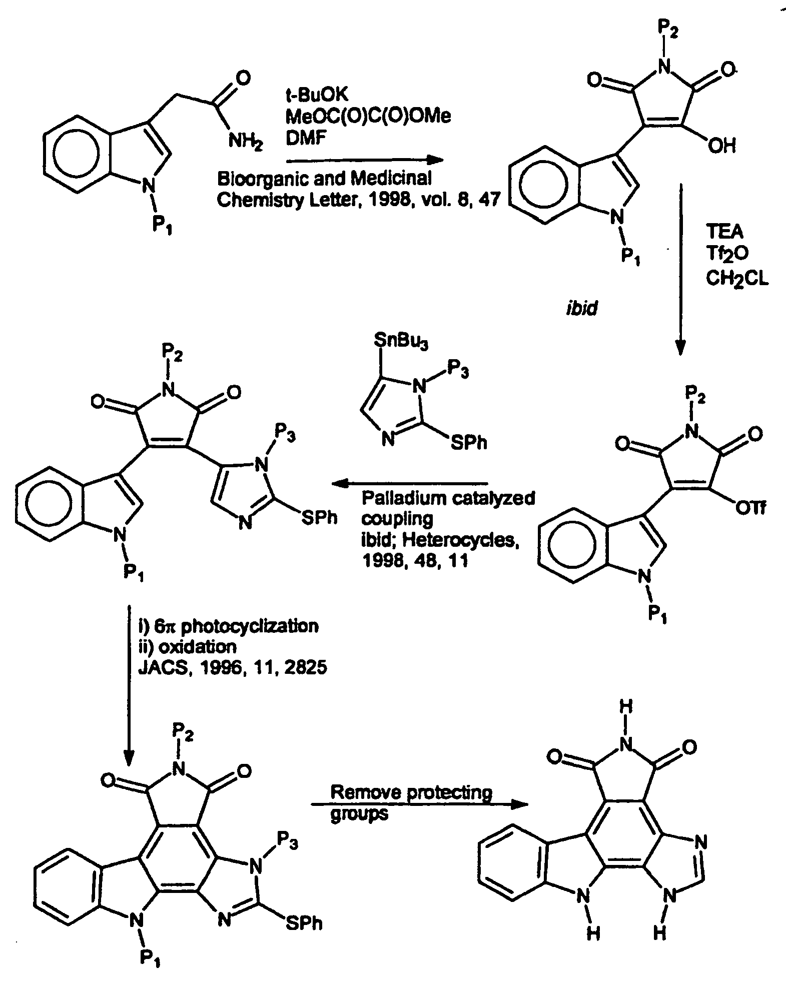

An alternative synthesis scheme, outlined below, involves essentially a single coupling step to produce a didemnimide compound followed by cyclization to produce a compound of Formula I, wherein F, F', X and Y are as defined for Formula I, and W is as defined for Formula I joined to the reactant at (a) and not (b):

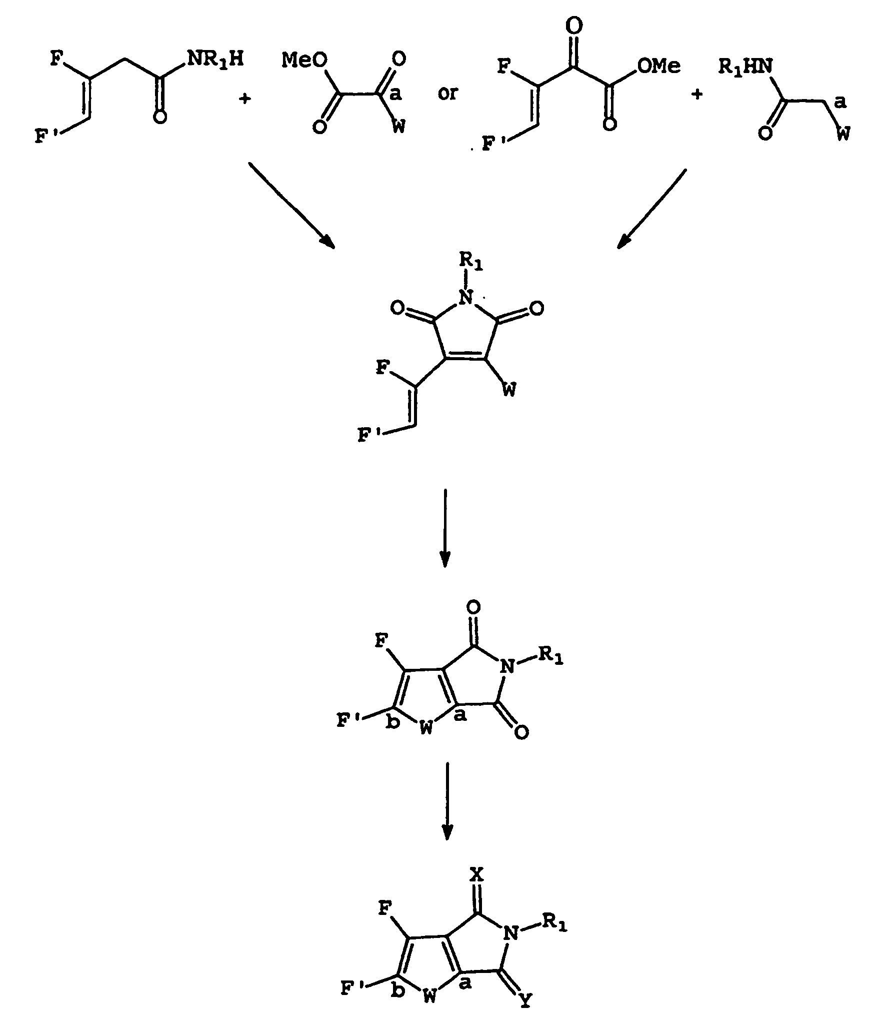

The above-described preferred synthesis scheme is adapted for production of granulatimide and isogranulatimide (typically produced in about 9:1 ratio); as described below. Compound **8** may be produced as described in Example 2 below. With this embodiment of the preferred synthesis scheme, condensation of **8** with the indole-3-acetamide (**9**) should be carried out in the presence of molecular sieves, otherwise a second major product results from displacement of the phenylthio group on (**10**) by a methoxyl moiety.

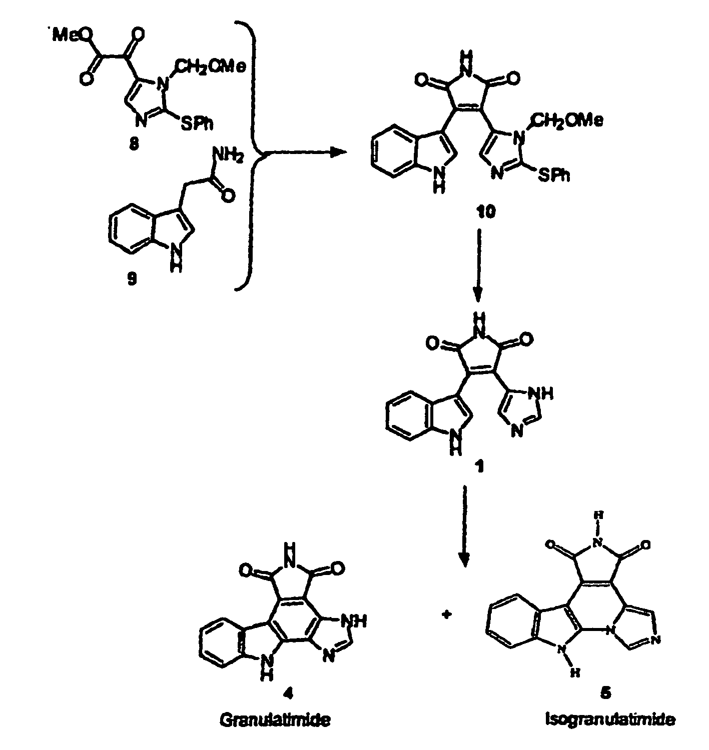

Alternatively, compounds of Formula I can be produced from granulatimide or isogranulatimide which may be synthesized as described above or obtained from natural sources as described in the Examples. The following schematic describes modification of granulatimide to produce derivatives having different X and Y substituents. The ratio of II to III will be dictated by the choice of protecting groups P₁, and P₂ or P₃, which may be (for example) Cbz, R or H. A protecting group will be used at either P₂ or P₃, depending upon the position of the double bond in the ring. The chemistry is described in Link, *et al.* (1996) J. American Chemical Society 118:2825, at p. 2832.

All Z and R groups can be added to aromatic carbons in W and Ar₁ prior to the coupling step to prepare the maleimide intermediates. All R, RCO, Ar₂CO, and Ar₂CH₂ groups can be added to nitrogen atoms using base and an appropriate alkyl halide (RX), acyl halide (RCOX), anhydride ((RCO)₂₀) or benzyl halide (Ar₂CH₂X).

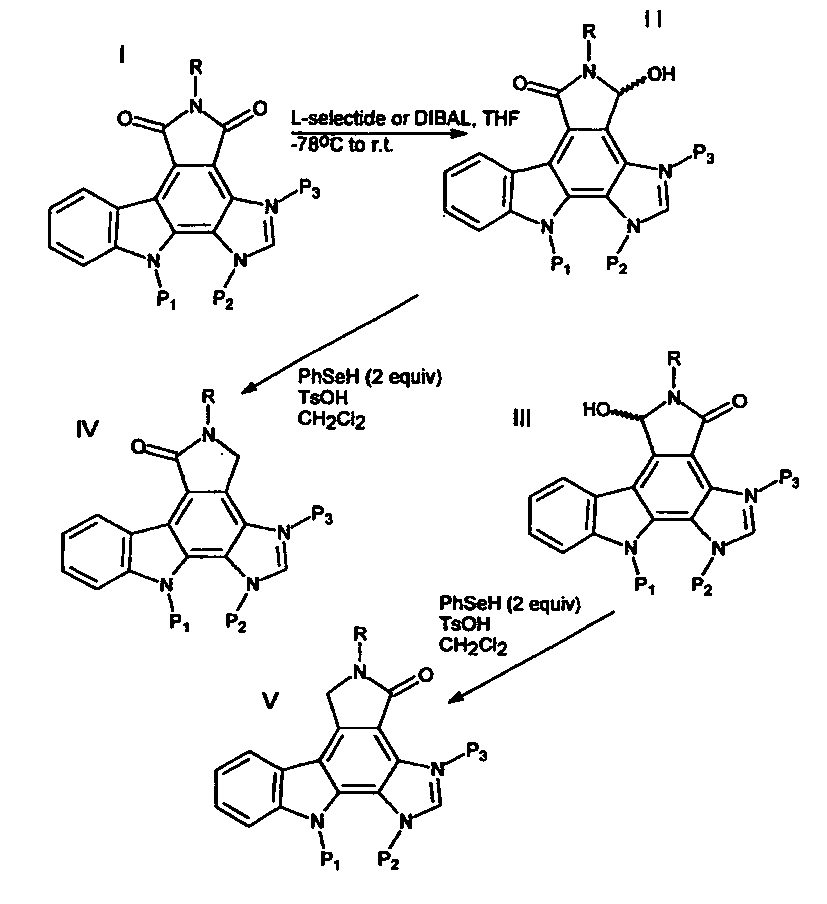

### PHARMACEUTICAL FORMULATIONS

The compounds of this invention can be incorporated into a variety of formulations for therapeutic administration. More particularly, the compounds of the present invention can be formulated into pharmaceutical compositions by combination with appropriate pharmaceutically acceptable carriers or diluents, and may be formulated into preparations in solid, semi-solid, liquid or gaseous forms, such as tablets, capsules, powders, granules, ointments, solutions, suppositories, injections, inhalants, gels, microspheres, and aerosols. As such, administration of the compounds can be achieved in various ways, including oral, buccal, rectal, parenteral, intraperitoneal, intradermal, transdermal, intracheal, etc., administration. The active agent may be systemic after administration or may be localized by the use of regional administration, intramural administration, or use of an implant that acts to retain the active dose at the site of implantation.

In pharmaceutical dosage forms, the compounds may be administered in the form of their pharmaceutically acceptable salts. They may also be used in appropriate association with other pharmaceutically active compounds. The following methods and excipients are merely exemplary and are in no way limiting.

For oral preparations, the compounds can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The compounds can be formulated into preparations for injections by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The compounds can be utilized in aerosol formulation to be administered via inhalation. The compounds of the present invention can be formulated into pressurized acceptable propellants such as dichlorodifluoromethane, propane, nitrogen and the like.

Furthermore, the compounds can be made into suppositories by mixing with a variety of bases such as emulsifying bases or water-soluble bases. The compounds of the present invention can be administered rectally via a suppository. The suppository can include vehicles such as cocoa butter, carbowaxes and polyethylene glycols, which melt at body temperature, yet are solidified at room temperature.

Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more compounds of the present invention. Similarly, unit dosage forms for injection or intravenous administration may comprise the compound of the present invention in a composition as a solution in sterile water, normal saline or another pharmaceutically acceptable carrier.

Implants for sustained release formulations are well-known in the art. Implants are formulated as microspheres, slabs, etc. with biodegradable or non-biodegradable polymers. For example, polymers of lactic acid and/or glycolic acid form an erodible polymer that is well-tolerated by the host. The implant containing granulatimide compounds is placed in proximity to the site of the tumor, so that the local concentration of active agent is increased relative to the rest of the body.

The term "unit dosage form", as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the novel unit dosage forms of the present invention depend on the particular compound employed and the effect to be achieved, and the pharmacodynamics associated with each compound in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

The combined use of granulatimide compounds and other cytotoxic agents has the advantages that the required dosages for the individual drugs is lower, and the effect of the different drugs complementary. Depending on the patient and condition being treated and on the administration route, the granulatimide compounds may be administered in dosages of 0.1 µg to 10 mg/kg body weight per day. The range is broad, since in general the efficacy of a therapeutic effect for different mammals varies widely with doses typically being 20, 30 or even 40 times smaller (per unit body weight) in man than in the rat. Similarly the mode of administration can have a large effect on dosage. Thus for example oral dosages in the rat may be ten times the injection dose. Higher doses may be used for localized routes of delivery.

A typical dosage may be a solution suitable for intravenous administration; a tablet taken from two to six times daily, or one time-release capsule or tablet taken once a day and containing a proportionally higher content of active ingredient, etc. The time-release effect may be obtained by capsule materials that dissolve at different pH values, by capsules that release slowly by osmotic pressure, or by any other known means of controlled release.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

For use in the subject methods, the granulatimide compounds may be formulated with other pharmaceutically active agents, particularly other anti-metastatic, anti-tumor or anti-angiogenic agents. Angiostatic compounds of interest include angiostatin, endostatin, carboxy terminal peptides of collagen alpha (XV), *etc*. Cytotoxic and cytostatic agents of interest include adriamycin, alkeran, Ara-C, BICNU, busulfan, CNNU, cisplatinum, cytoxan, daunorubicin, DTIC, 5-FU, hydrea, ifosfamide, methotrexate, mithramycin, mitomycin, mitoxantrone, nitrogen mustard, velban, vincristine, vinblastine, VP-16, carboplatinum, fludarabine, gemcitabine, idarubicin, irinotecan, leustatin, navelbine, taxol, taxotere, topotecan, etc.

### METHODS OF USE

The subject granulatimide compounds are administered to a subject having undesirable kinase activity, or conditions resulting therefrom. The compounds may also be administered to a subject in order to inhibit the G2 checkpoint, particularly in association with treatment of cancer cells, more particularly in combination with cytotoxic therapy directed at said cancer cells; e.g. radiation treatment, chemotherapeutic drugs, *etc*.

The host, or patient, may be from any mammalian species, *e.g*. primate *sp.*, particularly humans; rodents, including mice, rats and hamsters; rabbits; equines, bovines, canines, felines; *etc*. Animal models are of interest for experimental investigations, providing a model for treatment of human disease.

Tumors known to be susceptible to DNA damaging agents include carcinomas, *e.g.* colon, prostate, breast, ductal, endometrial, stomach, dysplastic oral mucosa, invasive oral cancer, non-small cell lung carcinoma, transitional and squamous cell, urinary carcinoma, *etc.*; neurological malignancies, *e.g.* neuroblastoma, gliomas, *etc.;* hematological malignancies, *e.g.* childhood acute leukaemia, non-Hodgkin's lymphomas, chronic lymphocytic leukaemia, malignant cutaneous T-cells, mycosis fungoides, non-MF cutaneous T-cell lymphoma, lymphomatoid papulosis, T-cell rich cutaneous lymphoid hyperplasia, bullous pemphigoid, discoid lupus erythematosus, lichen planus, etc.; and the like.

A combined therapy of granulatimide compounds and cytotoxic agents are administered to a host suffering from a susceptible tumor. Administration may be topical, localized or systemic, depending on the specific disease. The compounds are administered at a combined effective dosage that over a suitable period of time substantially reduces the tumor cell burden, while minimizing any side-effects, usually killing at least about 25% of the tumor cells present, more usually at least about 50% killing, and may be about 90% or greater of the tumor cells present. It is contemplated that the composition will be obtained and used under the guidance of a physician for *in vivo* use. To provide the synergistic effect of a combined therapy, the active agents can be delivered together or separately, and simultaneously or at different times within the day.

The susceptibility of a particular tumor cell to killing with the combined therapy may be determined by *in vitro* testing. Typically a culture of the tumor cell is combined with a combination of a cytotoxic compound and a granulatimide compound at varying concentrations for a period of time sufficient to allow the active agents to induce cell death, usually between about one hour and one week. For *in vitro* testing, cultured cells from a biopsy sample of the tumor may be used. The viable cells left after treatment are then counted.

The dose will vary depending on the specific cytotoxic agent utilized, type of tumor, patient status, *etc.*, at a dose sufficient to substantially ablate the tumor cell population, while maintaining patient viability. In some cases therapy may be combined with stern cell replacement therapy to reconstitute the patient hematopoietic function. Treatment will generally be continued until there is a substantial reduction, *e.g*. at least about 50%, decrease in the tumor burden, and may be continued until there are essentially no tumor cells detected in the body.

All publications discussed above and throughout the text are provided solely for their disclosure prior to the filing date of the present application. Nothing herein is to be construed as an admission that the inventors are not entitled to antedate such disclosure by virtue of prior invention.

The following examples are put forth so as to provide those of ordinary skill in the art with a complete disclosure and description of how to make and use the subject invention. Efforts have been made to ensure accuracy with respect to the numbers used (*e.g.* amounts, temperature, concentrations, *etc*.) but some experimental errors and deviations should be allowed for. Unless otherwise indicated, parts are parts by weight, molecular weight is average molecular weight, temperature is in degrees centigrade; and pressure is at or near atmospheric.

### EXAMPLES

### Example 1

### G2 Checkpoint Inhibition Assay.

MCF-7 mp53 cells (mutant for p53) were cultured as monolayers in DMEM supplemented with 10% fetal bovine serum, 2 mM L-glutamine, 50 units/ml penicillin, 50 µg/ml streptomycin, 1 mM sodium pyruvate, MEM non-essential amino acids, 1 µg/ml bovine insulin, 1 µg/ml hydrocortisone, 1 ng/ml human epidermal growth factor, and 1 ng/ml β-estradiol at 37°C in humidified 5% CO₂. The cells were seeded at 10,000 cells per well of 96-well polystyrene tissue culture plates (Falcon) in 100 µl medium. The cells were allowed to grow for 24 hours and then were irradiated with 6.5 Gy using a ⁶⁰Co source (Gammacell 200^{™}, Atomic Energy Commission of Canada) delivering γ-rays at a dose rate of 1.4 Gy/min.

Extracts from marine organisms at about 10µg/ml (from 1000-fold stocks in DMSO) and 100 ng/ml nocodazole (Sigma, from a 1000-fold stock in DMSO) were added 16 hours after irradiation and the cells were incubated for a further 8 hours. Caffeine at 2 mM (Sigma, from a 100 mM solution in phosphate-buffered saline) was used as a positive control.

After drug treatment, the cell culture medium was removed and the cells were lysed by adding 100 µl of ice-cold lysis buffer (1 mM EGTA pH 7.4, 0.2 mM PMSF) and pipeting up-and-down 10 times. The cell lysates were transferred to 96-well PolySorp^{™} plates (Nunc) and dried completely by blowing warm air at about 37°C with a hair dryer positioned about 3 feet above plates. Protein binding sites were blocked by adding 200 µl per well of antibody buffer (10 mM Tris HCl pH 7.4, 150 mM NaCl, 0.1 mM PMSF, 3% (w/v) dried non-fat milk (Carnation)) for 1 hour at room temperature. This was removed and replaced with 100 µl antibody buffer containing 0.1-0.15 µg/ml TG-3 monoclonal antibody and horseradish peroxidase-labelled goat anti-mouse IgM (Southern Biotechnology Associates) at a dilution of 1/500.

After overnight incubation at 4°C, the antibody solution was removed and the wells were rinsed 3 times with 200 µl 10 mM Tris HCl pH 7.4, 0.02% Tween 20^{™}. 100 µl of 120 mM Na₂HPO₄, 100 mM citric acid, pH 4.0 containing 0.5 mg/ml 2,2'-azino-bis (3-ethylbenzthiazoline-6-sulfonic acid) and 0.01% hydrogen peroxide was added for 1 hour at room temperature and the plates were read at 405 nm using a BioTek^{™} plate reader. Positive controls treated with 2 mM caffeine gave absorbance readings of about 1.0.

G2 checkpoint inhibition was detected in extracts from the ascidian *Didemnum granulatum* (Subphylum Urochordata, Class Ascidiacea) collected from rocky, shallow water marine habitats along the coastline of southern Brazil.

### Collection and Extraction of D. Granulatum.

*Didemnum granulatum* (85 g wet wt.) was collected during the summer of 1995 at depths of 1 m at the rocky shore of Araca beach, Sao Sebastiao (Sao Paulo state, southeastern Brazil). A second collection was made at the Arquipelago do Arvoredo (Santa Catarina state, southern Brazil, 150 g wet wt.) and in the Sao Sebastiao Channel (Sao Paulo state, 185 g wet wt.) during November 1997. Freshly collected animals were stored in ethanol at -20°C. Animals obtained from the different collections were processed separately as follows: after decantation of ethanol, the animal was blended and extracted three times with methanol. The ethanol and methanol extracts were combined, filtered and evaporated *in vacuo* to give a gummy residue. The bulk of the residue was dissolved in 8:2 MeOH-CH₂Cl₂ and filtered for elimination of inorganic salts while small amounts of the residue were dissolved in DMSO for use in the G2 checkpoint inhibition assay described above.

### Isolation of Active Compounds.

Methanol extracts from the Brazilian ascidian *D. granulatum* were fractionated by gel filtration on Sephadex LH-20 (eluent: MeOH) followed by reversed phase HPLC (eluent: acetonitrile/0.05% trifluoroacetic acid (1:1)) affording a series of eight fractions having different constituents by TLC. Only one fraction exhibited G2 checkpoint inhibition activity, which provided the novel alkaloid compounds later termed iso-granulatimide and granulatimide. Other fractions contained various forms of the alkaloid didemnimide which were inactive. Didemnimide A and D were identified by comparison of NMR and MS data with literature values (Vervoort, H.C. *et al. [supra]*).

NMR experiments using a compound isolated from the active fraction identified a disubstituted imidazole moiety. The compound differed from Didemnimide A by the loss of two hydrogen atoms. This led to the conclusion that the new compound has a bond between C-2 of the indole and ether C-14 or N-17 of the imidazole fragment of Didemnimide A. The presence of an anisoptropic effect in the novel compound and not in didemnimide A indicated a rigid planar heterocycle, unlike didemnimide A in which the indole and maleimide rings are twisted relative to each other along the C-3 to C-8 bond. The heterocyclic aromatic core of the novel compound is without precedent in natural products.

### Characterization of Isogranulatimide and Granulatimide.

NMR data were collected on Bruker WH 400 (¹H NMR, COSY and NOE), AM 400 (¹³C NMR) and AMX 500 (HMBC and HMQC) spectrometers. Proton spectra were referenced using the internal residual signal of DMSO-*d₅* (δ 2.49) and carbon spectra were referenced to the DMSO methyl resonance (δ 39.5). FABMS data were collected on a Kratos concept IIHQ hybrid mass spectrometer with cesium ion secondary ionization and magnetic sector mass analyzer. Samples were dissolved in a MeOH-thioglycerol matrix, and spectra were obtained using a source voltage of 8 kV and a cesium ion gun voltage of 12 kV. Infrared spectra were recorded on a Perkin Elmer 1710 spectrophotometer and UV spectra on a spectrophotometer.

A compound (later termed isogranulatimide) was isolated from active fraction as a red amorphous solid (UV (λₘₐₓnm(ε)) 210 (10,200), 231 (10,600) 280 (6,550), 470 (1,870)) that gave a [M + H]+ion at m/z 277.0730 in the HRFABMS appropriate for a molecular formula of C₁₅H₈N₄O₂ (calculated mass for C₁₅H₈N₄O₂, 277.0714). An NH proton resonance at δ 11.11 showed HMBC correlations to carbonyl resonances at δ 169.8 and 168.8 and aromatic carbon resonances at δ 126.39 and 112.22, confirming the presence of a maleimide substructure. The COSY spectrum identified a four proton spin system (δ 8.51, d, J = 7 Hz (H-4)); 7.43, dd, J = 7,7 Hz (H-6); 7.35, dd, J = 7,7 Hz (H-5); 7.67 d, J= 7 (H-7) that may be assigned to the H-4 to H-7 protons of an indole residue. Irradiation of a broad proton resonance at δ 13.48 induces an NOE only in the aromatic doublet at δ 7.68, which assigns the doublet to H-7 and the broad proton resonance to the indole NH. The absence of a resonance in the ¹H NMR spectrum of the compound that may be assigned to the indole H-2 or H-3 protons indicated the presence of substituents at C-2 and C-3.

Very broad resonances at δ 8.10 and δ 9.12 in the ¹H NMR spectrum were assigned to the imidazole moiety. The broadness of the imidazole resonances was attributed to tautomeric equilibrium of the NH protons. The large chemical shift observed for the H-4 resonance (δ 8.51) in the new compound relative to the chemical shift observed for the H-4 resonance in didemnimide A (δ 7.07) was attributed to a neighbouring group effect from a C-9 maleimide carbonyl.

As described above, the new compound was predicted to be a polycyclic aromatic differing from didemnimide A by the presence of a bond between the C-2 carbon of the indole fragment and either C-14 or N-17 of the imidazole fragment. In the former case, the compound would be granulatimide as described herein and in the latter case, iso-granulatimide. The broadness of the imidazole resonances in the NMR spectra initially made use of NMR ineffective in distinguishing the two compounds. Therefore, both granulatimide and isogranulatimide were synthesized as described herein and compared to the compound derived from the natural source which revealed the compound to be isogranulatimide. TLC analysis (CH₂Cl₂ - MeOH 9:1; UV at 254nm) of pooled active fractions from the natural extract using synthetic granulatimide as a reference revealed presence of the latter compound in an amount substantially less than isogranulatimide. However, it was determined that the synthetic granulatimide is very insoluble in most common organic solvents including ethanol and methanol and was therefore not efficiently extracted from the natural source in the procedure described above.

Granulatimide is soluble in DMF or DMSO and one of the latter solvents is recommended for extraction of granulatimide from the natural source.

Table 1 provides comparative ¹H-NMR data determined using synthetic compounds as recorded in DMSO-*d₆.*

**Table 1**

| Atom No. | Granulatimide (**4**) | Isogranulatimide (**5**) |
|---|---|---|
| 1 | 12.58,bs | 13.48,bs |
| 2 | - | - |
| 4 | 8.89,d,J=7Hz | 8.51,d,J=7Hz |
| 5 | 7.30,dd,J=7.7Hz | 7.35,dd,J=7,7Hz |
| 6 | 7.48,dd,J=7,7Hz | 7.43,dd,J=7,7Hz |
| 7 | 7.61,d,J=7Hz | 7.67,d,J=7Hz |
| 10 | 10.96,s | 11.11,bs |
| 14 | - | 8.10,b |
| 16 | 8.50,s | 9.12,b |
| 17 | 13.57,s | - |
| 18 | - | - |

### Activity of Isogranulatimide and Granulatimide.

Isogranulatimide shows half-maximal G2 checkpoint inhibition (IC₅₀) at 1.8±0.2 µM (Figure 1A). Didemnimide A shows no activity at concentration of 0.1-30 µM). Isogranulatimide shows mild cytotoxicity, with an IC₅₀ of 40±4 µM (Fig. 1B, curve 0 Gy), well above the IC₅₀ for checkpoint inhibition.

G2 checkpoint inhibitors and DNA-damaging agents would be expected to kill p53- cells synergistically. When such cells were exposed to 2, 4 or 6 Gy of γ-irradiation and to different concentrations of isogranulatimide for 16 hours, cells died in higher numbers than for the sum of each treatment alone (Figure 1B), showing that γ-irradiation and isogranulatimide acted synergistically.

The IC₅₀ for cytotoxicity of isogranulatimide was reduced by 5-fold when cells were irradiated with 6 Gy (Figure 1B). By comparison, caffeine, the most commonly used G2 checkpoint inhibitor, exhibits an IC₅₀ of 1 mM for G2 checkpoint inhibition, has very little cytotoxicity alone and also acts synergistically with γ-irradiation.

Irradiation with isogranulatimide treatment does not kill p53+ cells synergistically, as shown in Figure 1C. Therefore, the isogranulatimide preferentially kills irradiated p53- cells over irradiated p53+ cells, suggesting it would preferentialliy kill p53- tumors in humans treated with DNA damaging therapy.

As is shown in Figure 2, granulatimide may be more potent as a G2 checkpoint inhibitor (IC₅₀ = 1.3µm) as compared to isogranulatimide.

### Example 2

### Synthesis of Isogranulatimide and Granulatimide.

Throughout these examples, TLC was carried out by using commercial aluminum-backed silica gel 60 plates. Flash chromatography was carried out with 230-400 mesh silica gel (E. Merck). THF was distilled from sodium/benzophenone and CH₂Cl₂ was distilled from CaH₂. Commercial EtOH (reagent grade) and MeCN (HPLC grade) were used without further purification. Molecular sieves were dried under vacuum with heating for 5 hours prior to use. All reactions were carried out under an atmosphere of dry argon using glassware that had been thoroughly flame dried.

### Methyl 2-(1-methoxymethyl-2-phenylthioimidazol-5-yl)glyoxylate (8).

To a cold (-78°C), stirred solution of 1-methoxymethyl-2-phenylthioimidazole (Ohta, S. *et al.* (1992) Chem. Pharm. Bull. 40:2681) (340 mg, 1.55 mmol) in dry THF (8.0 mL) was added a solution of n-BuLi (1.47 M in hexanes, 1.26 mL, 1.85 mmol) and the mixture was stirred for 1 hour. A solution of dimethyl oxalate (540 mg. 4.58 mmol) in dry THF (2.0 mL) was added and the mixture was stirred at -78°C for an additional 1.25 hours. The reaction mixture was treated with saturated aqueous NH₄Cl (5.0 mL) and Et₂O (20 mL). The phases were separated and the aqueous layer was extracted with Et₂O (25 mL). The combined organic extracts were washed (brine, 10 mL), dried (MgSO₄), and concentrated. Flash chromatography (35 g of silica gel, 1:1 petroleum ether-Et₂O) of the crude material afforded 305 mg (66%) of 8 as a beige solid that exhibited mp 59-60 °C; IR (KBr) 1729,1657,1305,1273,1167,1114, 784 cm⁻¹; ¹H NMR (CDCl₃, 400 MHz) δ 8.23 (s, 1 H), 7.52-7.54 (m. 2 H), 7.37-7.38 (m, 3 H), 5,81 (s, 2 H), 3.91 (s, 3 H), 3.36 (s, 3 H); ¹³C NMR (CDCl₃, 100 MHz) δ 172.2, 161.6, 154.1, 145.4, 145.3, 133.2, 129.5, 129.2, 128.7, 75.8, 56.5, 53.0; HREIMS calcd for C₁₄H₁₄N₂O₄S 306.0674, found 306.0674.

### 3-[4-(1-methoxymethyl-1H-2-phenylthio-imidazol-5-yl)-2,5-dioxo-2,5-dihydro-1H-pyrrol-3-yl]-indole (10).

To a stirred solution of t-BuOK (150 mg, 1.34 mmol) in DMF (3.0 mL) at room temperature was added sequentially 4Å molecular sieves (~500 mg) and a solution of **8** (167 mg, 0.54 mmol) and indole-3-acetamide (9) (114 mg, 0.66 mmol) in DMF (4.0 mL). the reaction mixture was heated to 45°C and stirred for 12 hours. The dark purple solution was treated with hydrochloric acid (1 N, 3.0 mL) and EtOAc (30 mL). The phases were separated and the aqueous layer was extracted with EtOAc (2 x 10 mL). The combined organic extracts were washed (brine 4 x 10 mL), dried (MgSO₄), and concentrated. Flash chromatography (12 g of silica gel, 30:1 CH₂Cl₂-MeOH) of the crude material afforded 208 mg (90%) of 10 as an orange solid that exhibited mp 243°C; IR (KBr) 3400-2600, 1765, 1708,1537, 1341, 741 cm⁻¹; ¹H NMR (400 MHz) δ 12.11 (br s, 1 H), 11.25 (br s, 1 H), 8.14 (s, 1 H) 7.46 (d, 1 H, J = 7.9 Hz), 7.18-7.33 (m, 6 H), 7.14 (poorly resolved dd, 1 H), 6.77 (poorly resolved dd, 1 H), 6.51 (d, 1 H, J = 8.0), 500 (s, 2 H), 2.97 (s, 3 H); ¹³C NMR (100 MHz) δ 171.7, 171.6, 140.3. 136.7, 135.3, 133.8, 133.5, 132.5, 129.5, 128.4, 127.2, 125.7, 124.3, 122.7, 120.8, 120.2, 117.4, 112.6, 104.6, 75.8, 55.9; HREIMS calcd for C₂₃H₁₈N₄O₃S 430.1099, found 430.1099.

### 3-[4-(3-methoxymethyl-3H-imidazol-4-yl)-2,5-dioxo-2,5-dihydro-1H-pyrrol-3-yl]-indole (11).

To a refluxing solution of **10** (52 mg, 0.12 mmol) in EtOH (5.0 mL) was added Raney Ni (W-2, ~150 mg) and the suspension was refluxed for 1 hour. An additional amount of Raney Ni (W-2, ~100 mg) was added and the mixture was refluxed for an additional 3 hours. The reaction mixture was then cooled to room temperature and filtered through Celite. The Celite was washed with CH₂Cl₂-MeOH (1:1, 75 mL) and the combined filtrate was concentrated. Flash chromatography (14 g of silica gel, 20:1 CH₂Cl₂-MeOH) of the crude material afforded 33 mg (85%) of 11 as an orange solid: m.p.> 235°C, dec.; IR (KBr) 3400-2600, 1765, 1703, 1537, 1440, 1342, 1113, cm⁻¹; ¹H NMR (400 MHz) δ 10.89 (br s, 1 H), 10.03 (br s, 1 H), 8,07 (s, 1 H), 7.94 (s, 1 H), 7.44 (d, 1 H, J = 8.5 Hz), 7.09 (poorly resolved dd, 1 H), 7.0 (s, 1 H), 6.79 (poorly resolved dd, 1 H), 6.46 (d, 1 H, J = 8.0 Hz), 5.02 (s, 2 H), 3.07 (s, 3 H); ¹³C NMR (100 MHz) δ 171.8, 171.7, 140.5, 136.5, 134.3, 132.9, 131.8, 124.3, 122.4, 121.7, 120.5, 120.3, 118.4, 112.3, 104.7, 76.4, 55.6; HREIMS calcd for C17H14N4O3 322.1066, found 322.1066.

### Synthesis of Didemnimide A (1).

To a stirred suspension of **11** (67.4 mg, 0.209 mmol) in CH₂Cl₂ (15.0 mL) at room temperature was added a solution of BBr₃ in CH₂Cl₂ (1.0 M, 2.1 mL, 2.1 mmol) and the deep blue solution was stirred for 5 hours. The mixture was treated with saturated aqueous NaHCO₃ (10 mL) and EtOAc (20 mL) and then was stirred for 0.25 hours. The phases were separated and the aqueous layer was extracted with EtOAc (2 x 15 mL). The combined organic extracts were washed (brine, 10 mL), dried (MgSO₄) and concentrated. Flash chromatography (10 g of silica gel, 15:1 CH₂Cl₂-MeOH) of the crude material afforded 15.8 mg of recovered **11** as well as 45.0 mg (77%, 100% based on recovered starting material) of 1 as an orange solid ¹H NMR (400 MHz, major tautomer) δ 12.45 (br s, 1 H), 11.66 (br s, 1 H), 10,87 (br s, 1 H), 8.05 (s, 1 H), 7.71 (br s, 1 H), 7.68 (br s, 1 H), 7.39 (br d, 1 H, J = 7.8 Hz), 7.07 (br m, 2 H), 6.87 (br m, 1 H); ¹³C NMR (100 MHz) δ 172.8, 172.6, 136.1, 135.9, 130.9, 130.5, 126.0, 126.0, 121.7, 121.3, 119.7, 119.2, 112.2, 111.5, 105.0; HREIMS calcd for C₁₅H₁₀N₄O₂ 278.0804, found 278.0804.

### Granulatimide (4) and Isogranulatimide (5).

To a solution of **1** (12.9 mg, 0.046 mmol) in MeCN (5.0 mL) was added a catalytic amount of palladium-on-carbon (10% Pd) and the resulting mixture was sparged with argon for 0.5 hours. The mixture was irradiated (450 Watt Hanovia medium pressure mercury vapor lamp, quartz reaction vessel) for 6.5 hours. The reaction mixture was filtered through Celite, the Celite was washed with DMF (10 mL), and the combined filtrate was concentrated. The remaining material was taken up in EtOAc (30 mL) and the resultant solution was washed (brine, 4 x 20 mL), dried (MgSO₄) and concentrated. Flash chromatography (20 g of silica gel, 10:1 CH₂Cl₂-MeOH) of the crude material afforded 11.7 mg (91%) of 4 as a yellow solid and 1.0 mg (8%) of 5 as a red solid. Final purification was achieved on RPHPLC (1:1 CH₃CN-O.05% TFA). Granulatimide (**4**): ¹H NMR (400 MHz) see Table 1; UV (MeOH) 230, 270, 285, 295, and 380 nm; HREIMS calcd for C₁₅H₈N₄O₂ 276.0647, found 276.0652. Isogranulatimide (**5**): See above and Table 1.

### Example 3

### Alternative syntheses of Isogranulatimide (5).

*Method A:* To a stirred solution of didemnimide A (**1**) (32.6 mg, 0.117 mmol) in 2-butanone (7.0 mL) at rt is added sequentially 4Å molecular sieves (~100 mg) and CuCl₂ (79 mg, 0.59 mmol). The reaction mixture was stirred for 15 h at rt. The dark purple solution was treated with 10% solution of NaOH until the pH was neutral and diluted with EtOAc (30 mL). The phases were separated and the aqueous layer was extracted with EtOAc (5 x 10 mL). The combined organic extracts were washed with brine (10 mL), dried (MgSO₄) and concentrated. Flash chromatography (15 g of silica gel, 15:1 CH₂Cl₂-MeOH) of the crude material afforded 19.5 mg (60%) of recovered didemnamide A as well as 12.3 mg (38%, 95% based on recovered starting material) of isogranulatimide (5) as a purple solid.

*Method B:* A stirred solution of didemnimide A (**1**) (14.7 mg, 0.053 mmol) in DMSO (5.0 mL) was heated to 160 °C for 1 h. The reaction mixture was cooled to rt, diluted with EtOAc (30 mL), washed with brine (3 X 10 mL), dried (MgSO₄), and concentrated. Flash chromatography (25 g of silica gel, 10:1 CH₂Cl₂-MeOH) of the crude material afforded 12.4 mg (85%) of isogranulatimide (5) as a purple solid; IR (KBr) 2732, 1758, 1719, 1586, 1568, 1368, 1234, 1109, 744 cm⁻¹; ¹H NMR (400 MHz) δ 13.39 (bs, 1 H), 11.09 (bs, 1 H), 8.84 (s, 1 H), 8.44 (d, 1 H, *J =* 7.6 Hz), 7.82 (s, 1 H), 7.62 (d, 1 H, *J =* 8.0 Hz), 7.40 (dd, 1 H, *J*= 8,8 Hz), 7.31 (dd, 1 H, *J*= 8,8 Hz); ¹³C NMR (100 MHz) δ 169.9, 169.0, 135.6, 134.9, 126.9, 124.7, 123.1, 122.3, 121.8, 121.3, 121.0, 113.9, 112.3, 97.6; HREIMS calcd for C₁₅H₈N₄O₂ 276.0647, found 276.0647

### Example 4

### Synthesis of Granulatimide Compounds.

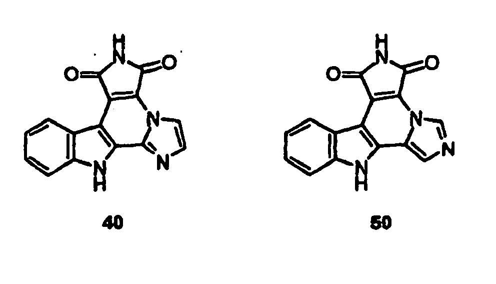

### Retrosynthesis

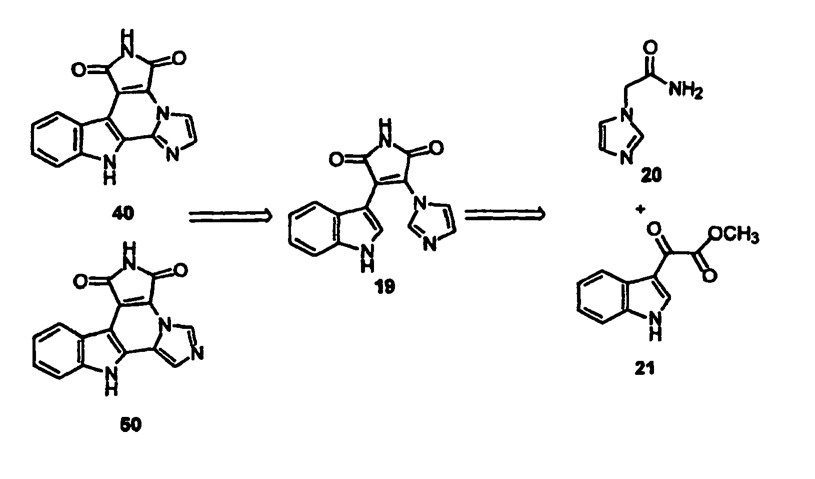

### Synthetic Scheme.

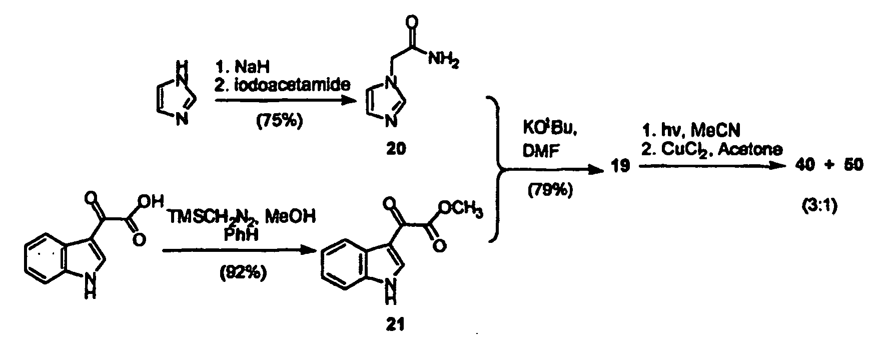

### Imidazol-1H-1-acetamide (20).

To a stirred suspension of NaH (60% dispersion in oil, 352 mg, 8.80 mmol) in DMF (5.0 mL) at 0°C was added a solution of imidazole (536 mg, 8.00 mmol) in DMF (10.0 mL). The reaction mixture was warmed to rt and stirred for 0.5 h. The reaction mixture was then cooled to 0°C and iodoacetamide (1.63 g, 8.81 mmol) was added in one portion. The resultant mixture was then warmed to rt and stirred for an additional 1.5 h. The reaction mixture was diluted with CH₂Cl₂ (20 mL) and Nal crystals were removed by filtration. The mother liquor was concentrated and placed on a neutral alumina column (40 g) and eluted with CH₂Cl₂-MeOH (6:1), and concentrated. Flash chromatography (60 g of silica gel, 7:1 CH₂Cl₂-MeOH) of the crude material afforded 747 mg (75%) of **20** as a white solid that exhibited mp 185-186 °C ; IR (KBr) 3349, 3116, 1685, 1516, 1408, 1312, 1236, 1080, 750, 663 cm⁻¹ ; ¹H NMR (400 MHz) δ 7.54 (s, 1 H), 7.51 (bs, 1 H). 7.20 (bs, 1 H), 7.06 (s, 1 H), 6.84 (s, 1 H), 4.61 (s, 2 H); ¹³C NMR (100 MHz) δ 168.5, 138.0, 127.5, 120.4, 48.4; HREIMS calcd for C₅H₇N₃O 125.0589, found 125.0589.

### Methyl Indol-3-ylglynxylate (21).

To a cold (0 °C) stirred solution of 3-indolegtyoxylic acid (1.00 g, 5.29 mmol) in MeOH (10.0 mL) and benzene (40.0 mL) was added (trimethylsilyl)diazomethane (2.0 M in hexanes, 5.3 mL, 10.6 mmol) and the mixture was warmed to rt and stirred for 4 h. The solvent was removed under vacuo to provide a beige solid. Flash chromatography (80 g of silica gel, 25:1 then 10:1 CH₂Cl₂-MeOH) of the crude material afforded 990 mg (92%) of **21** as a beige solid that exhibited: ¹H NMR (400 MHz) δ 12.40 (bs, 1 H), 8.44 (d, 1 H, J = 3.0 Hz), 8.15 (d, 1 H, J = 7.0 Hz), 7.54 (d, 1 H, J = 7.6 Hz), 7.24-7.31 (m, 2 H), 3.88 (s, 3 H); ¹³C NMR (100 MHz) δ 178.7, 163.9, 138.3, 136.7, 125.4, 123.8, 122.8, 121.1, 112.7, 112.4, 52.5; HREIMS calcd for C₁₁H₉NO₃ 203.0582, found 203.0582.

### 3-[4-(1H-imidazol-1-yl)-2,5-dioxo-2,5-dihydro-1H-pyrrol-3-yl]-indole (19).

To a stirred solution of *t*-BuOK (140 mg, 1.25 mmol) in DMF (2.0 mL) at 0 °C was added a solution of **20** (31.1 mg, 0.249 mmol) and methyl indole-3-glyoxylate (**21**) (101.1 mg, 0.498 mmol) in DMF (3.0 mL). The reaction mixture was stirred for 12 h at rt and then heated to 45 °C for 3 h. The reaction mixture was then cooled and treated with saturated aqueous NH₄Cl (5 mL) and EtOAc (20 mL). The phases were separated and the aqueous layer was extracted with EtOAc (2 x 20 mL). The combined organic extracts were washed with brine (4 x 10 mL), dried (MgSO₄), and concentrated. Flash chromatography (25 g of silica gel, 10:1 then 6:1 CH₂Cl₂-MeOH) of the crude material afforded 54.5 mg (79%) of **19** as a red solid that exhibited IR (KBr) 3121, 2729, 1767, 1719, 1651, 1495, 1340, 1239, 746 cm⁻¹; ¹H NMR (400 MHz) δ 10.75 (bs, 1 H), 10.00 (bs, 1 H), 8.03 (d, 1 H, *J* = 3.1 Hz), 7.78 (s, 1 H), 7.48 (d, 1 H, *J* = 8.2 Hz), 7.29 (s, 1H), 7.11 (dd, 1 H, *J* = 8,8 Hz), 7.04 (s, 1 H), 6.80 (dd, 1 H, *J* = 8,8 Hz), 6.21 (d, 1 H, *J* = 7.6 Hz); ¹³C NMR (100 MHz) δ 170.4, 168.4, 137.8, 136.4, 131.6, 128.8, 125.8, 124.3, 124.1, 122.5, 120.6, 120.1, 119.6. 112.3. 102.3; HREIMS calcd for C₁₅H₁₀N₄O₂ 278.0804, found 278.0804.

### RAB-009 (40) and RAB-010 (50).

A solution of **19** in t-BuOH and MeCN was sparged with argon for 0.5 h. The yellow mixture was then irradiated (450 Watt Hanovia medium pressure mercury vapour lamp, quartz reaction vessel) for 4 h. The reaction mixture was concentrated and redissolved in acetone (10 mL). To the crude reaction product in acetone at rt was added 10 X CuCl₂ and the reaction mixture was stirred, exposed to air, overnight. The solvent was then removed in vacuo and the residue was taken up in DMF (10 mL) and filtered through celite. The filtrate was then diluted with EtOAc (50 mL) and washed with brine (4 X 10 mL), dried (MgSO₄), and concentrated to afford 40 and 50 in a 3:1 ratio. Trituration of the crude solid with MeOH afford small amounts of RAB-009 (40) as an insoluble yellow solid; IR (KBr) 3184, 2931, 2692, 1756,1718, 1656, 1365, 1309, 1134, 730 cm⁻¹; ¹H NMR (400 MHz) δ 13.14 (bs, 1 H), 11.27 (bs, 1 H), 8.63 (d, 1 H, *J* = 8.1 Hz), 8.46 (s, 1 H), 7.90 (s, 1 H), 7.66 (d, 1 H, *J* = 8.0 Hz), 7.50 (dd, 1 H, *J* = 7,7 Hz), 7.36 (dd, 1 H, *J* = 8,8 Hz); HREIMS calcd for C₁₅H₈N₄O₂ 276.0647, found 276.0647. Unfortunately the MeOH washes could not be purified to provide significant quantities of RAB-010 (50) or RAB-009 (40). A small amount (approx. 0.5 mg, approx. 70% pure) of RAB-010 (50) was recovered through repeated coloumn chromatographies and submitted for bioassay. Compound 40 was found to be inactive, having an IC₅₀ >100 µM in the G2 assay; while compound 50 was active, having an IC₅₀ of 1 µM.

### Example 5

### Synthesis of 60.

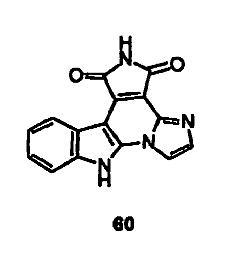

### Retrosynthesis.

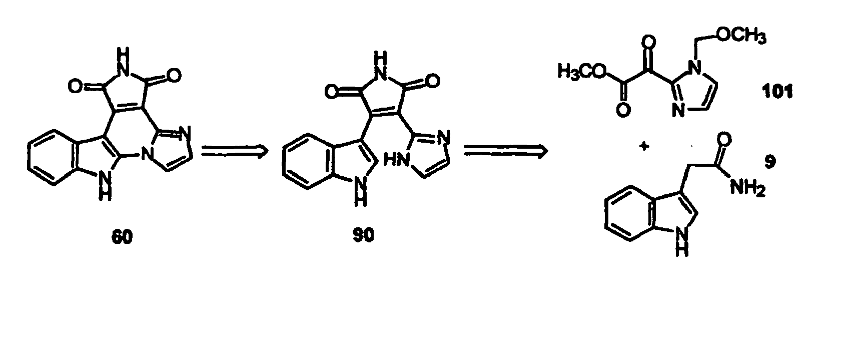

### Synthetic Scheme.

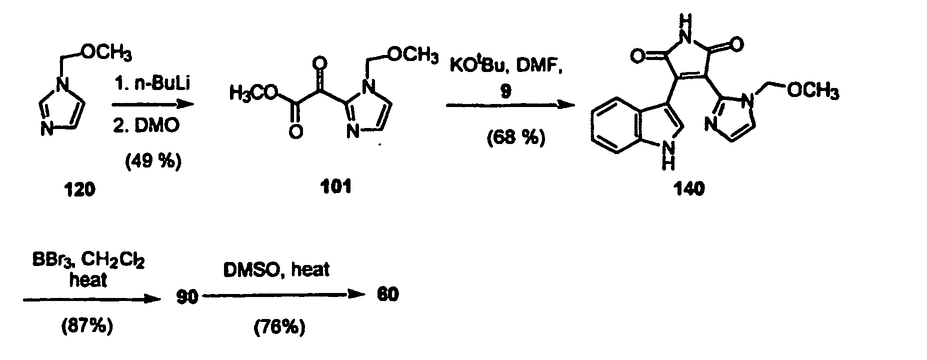

### Methyl 2-(1-methoxymethyl-1H-imidazol-2-yl)-glyoxylate (101).

To a cold (-48 °C), stirred solution of 1-methoxymethyl-1*H*-imidazole **(120)** (335 mg, 2.99 mmol) in dry THF (15.0 mL) was added a solution of *n*-BuLi (1.50 M in hexanes, 2.30 mL, 3.45 mmol) and the mixture was stirred for 1 h. The reaction mixture was then cooled to -78°C and added to a solution of dimethyloxalate (1.17 g, 9.9 mmol) in THF (10.0 mL) at -78°C. The mixture was stirred at -78°C for an additional 0.33 h. The reaction mixture was treated with saturated aqueous NH₄Cl (5 mL) and EtOAc (15 mL). The phases were separated and the aqueous layer was extracted with EtOAc (2 x 20 mL). The combined organic extracts were washed with brine (10 mL), dried (MgSO₄), and concentrated. Flash chromatography (50 g of silica gel, 50:1 CH₂Cl₂-MeOH) of the crude material afforded 290 mg (49%) of 101 as an oil that exhibited IR (KBr) 2957, 1744, 1679, 1412, 1276, 1215, 1109, 1005 cm⁻¹; ¹H NMR (CDCl₃, 400 MHz) δ 7.38 (s, 1 H), 7.33 (s, 1 H), 5.71 (s, 2 H), 3.96 (s, 3 H), 3.33 (s, 3 H); ¹³C NMR (CDCl₃, 100 MHz) δ 177.9, 164.3, 140.6, 132.9, 126.9, 78.8, 57.5, 53.4; HREIMS calcd for C₈H₁₀N₂O₄ 198.0641, found 198.0641.

*3-[4-(1-methoxymethyl-1H-imidazol-2-yl)-2,5-dioxo-2,5-dihydro-1H-pyrrol-3-yl]-indole (**140**).* To a stirred solution of 101 (142.0 mg, 0.717 mmol) and indole acetamide (62.7 mg. 0.360 mmol) in DMF (5.0 mL) was added a solution of t-BuOK (101 mg, 0.902 mmol) in DMF (5.0 mL) at rt. The reaction mixture was stirred overnight at rt then heated to 45 °C and stirred for an additional 12 h. The reaction mixture was then cooled and treated with saturated aqueous NH₄Cl (5 mL) and EtOAc (20 mL). The phases were separated and the aqueous layer was extracted with EtOAc (2 x 20 mL). The combined organic extracts were washed with brine (4 x 10 mL), dried (MgSO₄), and concentrated. Flash chromatography (40 g of silica gel, 40:1 CH₂Cl₂-MeOH) of the crude material afforded 79.1 mg (68 %) of 140 as a red solid that exhibited IR (KBr) 2500-3400, 1759, 1713, 1626, 1531, 1341, 1025, 745 cm⁻¹; ¹H NMR (400 MHz) δ 12.06 (bs, 1 H). 11.17 (bs, 1 H), 8.22 (s, 1 H), 7.45 (s, 1 H), 7.41 (d, 1 H, J = 7.9 Hz), 7.11 (s, 1 H), 7.08 (dd, 1 H, J = 8.1 Hz). 6.77 (dd, 1 H, J = 8,8 Hz), 6.23 (d, 1 H, J = 8.1 Hz), 5.06 (s, 2 H), 3.08 (s, 3 H); ¹³C NMR (100 MHz) δ 171.6, 171.4, 138.0, 137.7, 136.6, 132.7, 129.2, 124.8, 122.4, 122.0, 120.7, 120.2, 118.1, 112.1, 105.1, 76.8, 55.7; HREIMS calcd for C₁₇H₁₄N₄O₂ 322.1066, found 322.1066.

### 3-[4-(1H-imidazol-2-yl)-2,5-dioxo-2,5-dihydro-1H-pyrrol-3-yl]-indole (90).

To a stirred suspension of **140** (18.0 mg, 0.056 mmol) in CH₂Cl₂ (5.0 mL) at rt was added BBr₃ (1.0 M in CH₂Cl₂, 0.56 mL, 0.56 mmol) and the resulting blue mixture was heated under refluxing conditions for 5 h. The reaction mixture was cooled to rt and treated with saturated aqueous NaHCO₃ (5 mL) and EtOAc (10 mL) and then stirred for 0.5 h. The phases were separated and the aqueous layer was extracted with EtOAc (3 x 10 mL). The combined organic extracts were washed with brine (10 mL), dried (MgSO₄) and concentrated. Flash chromatography (25 g of silica gel, 20:1 CH₂Cl₂-MeOH) of the crude material afforded 13.5 mg (87%) of 90 as a red solid that exhibited IR (KBr) 3385, 3140, 1759, 1709, 1630, 1494, 1431, 1346, 1025, 1002, 747 cm⁻¹; ¹H NMR (major tautomer, 400 MHz) 812.32 (bs, 1 H), 11.90 (bs, 1H), 11.13 (bs, 1 H), 8.48 (d, 1 H, *J* = 3.2 Hz), 7.43 (d, 1 H, *J* = 8.0 Hz), 7.18-7.28 (m, 2 H), 7.10 (dd, 1 H, *J* = 7.7 Hz), 6.88-6.96 (m, 2 H); ¹³C NMR (100 MHz) δ 172.1, 172.0, 170.2, 137.1, 136.4, 133.0, 132.8, 125.2, 122.0, 121.6, 120.2, 120.0, 119.2, 111.9, 105.0; HREIMS calcd for C₁₅H₁₀N₄O₂ 278.0804, found 278.0804.

### RAB-007 (60).

A stirred solution of **90** (14.4 mg, 0.052 mmol) in DMSO (5 mL) was heated to 120 °C for 8 h. The reaction mixture was cooled to rt, diluted with EtOAc (30 mL), washed with brine (3 X 10 mL), dried (MgSO₄), and concentrated. Flash chromatography (20 g of silica gel, 20:1 then 10:1 CH₂Cl₂-MeOH) of the crude material afforded 11.0 mg (76%) of 60 as an orange solid that exhibited IR (KBr) cm⁻¹ 2600-3400, 1755, 1717, 1631, 1591, 1575, 1464, 1376, 1336, 1232,751; ¹H NMR (400 MHz) δ 13.36 (bs, 1 H), 11.13 (bs, 1 H), 8.63 (d, 1 H, *J* = 7.7 Hz), 8.40 (s, 1 H), 7.93 (s, 1 H), 7.69 (d, 1 H, *J* = 8.2 Hz), 7.48 (dd, 1 H. J = 7,7 Hz), 7.38 (dd, 1 H, J = 7,7 Hz); HREIMS calcd for C₁₅H₈N₄O₂ 276.0647, found 276.0647.

In the G2 checkpoint inhibition assay, compound **60** had an IC₅₀ of 2 µM.

### Example 6

### Inhibition of Protein Kinases by Granulatimide Compounds.

Granulatimide compounds as shown in Table 2, were assayed for their inhibition of G2 checkpoint, as previously described, and for inhibition of protein kinase activity against a panel of protein kinases, including CDK1; GSK3b and IL1.

The GSK3β peptide substrate used was YRRAAVPPSPSLSRHSSPHQS (PO₄H₂)EDEE-amide. The ILK1 substrate is CKRRRLASLR-amide.

The test compounds were diluted to 250 µM in 1% DMSO, then serially diluted using three-fold dilution steps. 100% DMSO was used as a negative control.

Kinase reactions are performed at room temperature. The reaction mixture contains diluted test compound, the protein kinase, substrate and γ-³²P ATP. The mixture is incubated for 15 minutes, and the reaction mix spotted, and counted. The results are shown in Table 2.

**Table 2**

| | IC₅₀ | | | |
|---|---|---|---|---|
| Granulatimide Compound | G2 checkpoint | CDK1 | GSK3b | ILK1 |
| 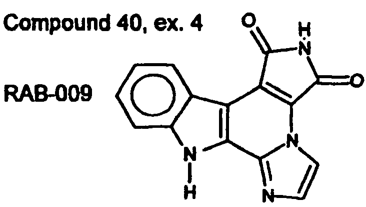 | >100 µM | >50 µM | 25 µM | >50 µM |
| 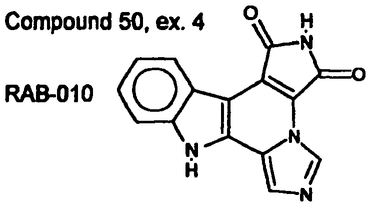 | 1 µM | 0.5 µM | 0.1 µM | 15 µM |
| 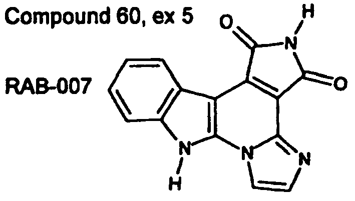 | 6 µM | 17 µM | 5 µM | >50 µM |
| Granulatimide, cmpd 4 | 1.3 µM | 2 µM | 2 µM | >50 µM |
| Isogranulatimide, cmpd 5 | 1.8 µM | 10 µM | 0.5 µM | >50 µM |
| UCN-01 (control) | n/d | 0.05 µM | 0.05 µM | >50 µM |
| staurosporine | n/d | <1 nM | 1 nM | >50 µM |

These results demonstrate that granulatimide compounds can be active as kinase inhibitors, in addition to their activity inhibiting the G2 checkpoint.

Although various aspects of the present invention have been described in detail, it will be apparent that changes and modification of those aspects described herein will fall within the scope of the appended claims.

## Claims

1. A compound having the structure: 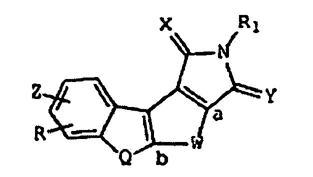 wherein:
W is selected from the group consisting of formula (i) or (ii) wherein the structures are as follows:
(i) 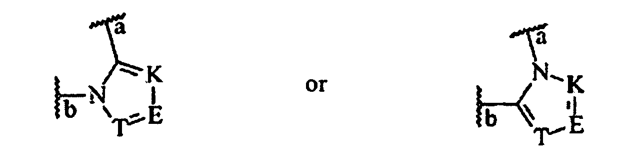 in which K, E and T are independently selected from the group consisting of: N, CR, and CZ, and wherein R and Z are as defined below; and
(ii) 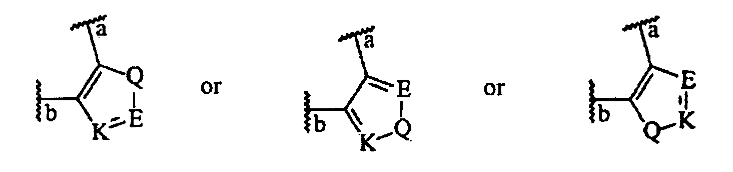
in which K and E are independently selected from the group consisting of: N, CR and CZ, and wherein R, Z and Q are as defined below;
R₁, is selected from the group consisting of: R; RCO-; Ar₂CO-; and, Ar₂CH₂-, wherein Ar₂ is an aromatic substituent selected from the group consisting of: phenyl, napthyl, anthracyl, phenanthryl, furan, pyrrole, thiophene, benzofuran, benzothiophene, quinoline, isoquinoline, imidazole, thiazole, oxazole, and pyridine, and Ar₂ may be optionally substituted with R or Z and R and Z may be defined as below;
R is selected from the group consisting of: H; and, a structural fragment having a saturated or unsaturated linear, branched, or cyclic, skeleton containing one to ten carbon atoms, in which the carbon atoms may be optionally substituted with a substituent selected from the group consisting of: -OH; -OR₃; -O₂CR₃, -SH; -SR₃; -SOCR₃; -NH₂; -NHR₃; -NH(R₃)₂; -NHCOR₃; NRCOR₃; -I; -Br; -Cl; -F; -CN; -CO₂H; -CO₂R₃; -CHO; -COR₃; -CONH₂; -CONHR₃; -CON(R₃)₂; -COSH; -COSR₃; -NO₂; -SO₃H; -SOR₃; and -SO₂R₃, wherein R₃ is a linear, branched or cyclic, one to ten carbon saturated or unsaturated alkyl group;
Z is an optional substituent selected from the group consisting of: H; -OH; -OR; -O₂CR; -SH; -SR; -SOCR; -NH₂; -NHR; -NH(R)₂; -NHCOR; NRCOR; -I; -Br; -Cl; -F; -CN; -CO₂H; -CO₂R; -CHO; -COR; -CONH₂; -CONHR; -CON(R)₂; -COSH; -COSR; -NO₂; -SO₃H; -SOR; and, -SO₂R:
Q is NH; and
X and Y are independently selected from the group consisting of: O; H; OH; and H₂.

2. The compound of claim 1, wherein R₁ is H or CH₃.

3. The compound of either claim 1 or claim 2, wherein X and Y are oxygen.

4. The compound of any one of claims 1 to 3, wherein W is a five membered ring of formula (i) or (ii) comprising at least one nitrogen atom; and wherein E, K and T, where present, are N or CH.

5. The compound of any one of claims 1 to 4 having the structure:

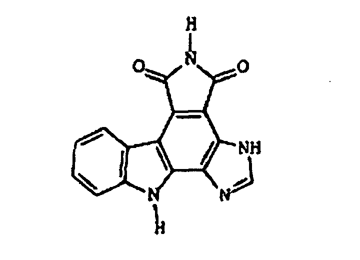

6. The compound of any one of claims 1 to 4 having the structure:

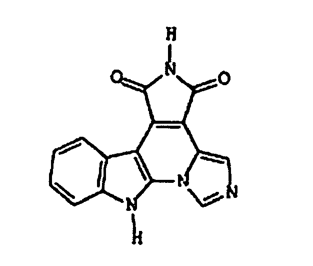

7. The compound of any one of claims 1 to 4 having the structure:

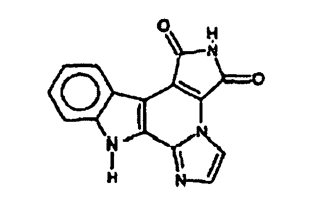

8. The compound of any one of claims 1 to 4 having the structure:

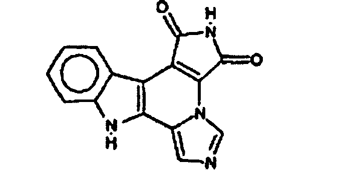

9. The compound of any one of claims 1 to 4 having the structure:

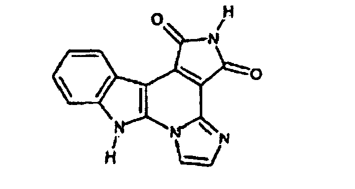

10. A pharmaceutical formulation comprising a compound of any one of claims 1-9, and a pharmaceutically acceptable carrier.

11. A compound of any one of claims 1 to 9 for use in a method of treatment of the human or animal body.

12. The use of a compound of any one of claims 1 to 9 in the manufacture of a medicament for the treatment of cancer.

13. The use of claim 12, wherein the medicament sensitizes cancer cells to the effects of DNA damaging agents on cancer cells.

## Patentansprüche

1. Verbindung mit der Struktur: 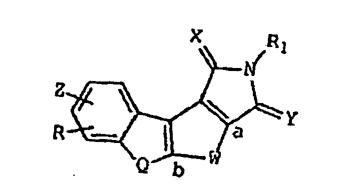 worin:
W aus der aus Formel (i) und (ii) bestehenden Gruppe ausgewählt ist, worin die Strukturen folgende sind:
(i) 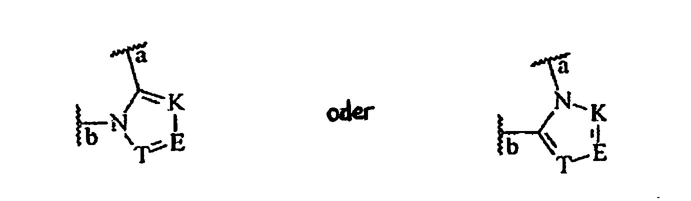 worin K, E und T jeweils unabhängig voneinander aus der aus Folgendem bestehenden Gruppe ausgewählt sind: N, CR und CZ, und worin R und Z wie nachstehend definiert sind; und
(ii) 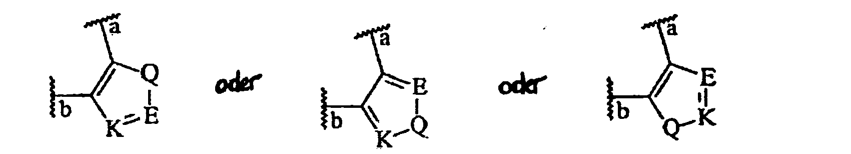
worin K und E jeweils unabhängig voneinander aus der aus Folgendem bestehenden Gruppe ausgewählt sind: N, CR und CZ, und worin R, Z und Q wie nachstehend definiert sind;
R₁ aus der aus Folgendem bestehenden Gruppe ausgewählt ist: R; RCO-; Ar₂CO-; und Ar₂CH₂-, worin Ar₂ ein aromatischer Substituent ist, der aus der aus Folgendem bestehenden Gruppe ausgewählt ist: Phenyl, Naphthyl, Anthracyl, Phenanthryl, Furan, Pyrrol, Thiophen, Benzofuran, Benzothiophen, Chinolin, Isochinolin, lmidazol, Thiazol, Oxazol und Pyridin, und Ar₂ gegebenenfalls mit R oder Z substituiert ist, worin R und Z wie nachstehend definiert sind;
R aus der aus Folgendem bestehenden Gruppe ausgewählt ist: H; und einem Strukturfragment mit einem gesättigten oder ungesättigten linearen, verzweigten oder zyklischen Skelett, das 1 bis 10 Kohlenstoffatome enthält, worin die Kohlenstoffatome gegebenenfalls mit einem Substituenten substituiert sind, der aus der aus Folgendem bestehenden Gruppe ausgewählt ist: -OH; -OR₃; -O₂CR₃, -SH; -SR₃; -SOCR₃; -NH₂; -NHR₃; -NH(R₃)₂; -NHCOR₃; -NRCOR₃; -I; -Br; -Cl; -F; -CN; -CO₂H; -CO₂R₃; -CHO; -COR₃; -CONH₂; -CONHR₃; -CON(R₃)₂; -COSH; -COSR₃; -NO₂; -SO₃H; -SOR₃; und -SO₂R₃, worin R₃ eine lineare, verzweigte oder zyklische, gesättigte oder ungesättigte Alkylgruppe mit 1 bis 10 Kohlenstoffen ist;
Z ein optionaler Substituent ist, der aus der aus Folgendem bestehenden Gruppe ausgewählt ist: H; -OH; -OR; -O₂CR; -SH; -SR; -SOCR; -NH₂; -NHR; -NH(R)₂; -NHCOR; -NRCOR; -I; -Br; -Cl; -F; -CN; -CO₂H; -CO₂R; -CHO; -COR; -CONH₂; -CONHR; -CON(R)₂; -COSH; -COSR; -NO₂; -SO₃H; -SOR; und -SO₂R;
Q NH ist; und
X und Y jeweils unabhängig voneinander aus der aus O; H, OH; und H₂ bestehenden Gruppe ausgewählt sind.

2. Verbindung nach Anspruch 1, worin R₁ H oder CH₃ ist.

3. Verbindung nach Anspruch 1 oder 2, worin X und Y Sauerstoff sind.

4. Verbindung nach einem der Ansprüche 1 bis 3, worin W ein fünfgliedriger Ring der Formel (i) oder (ii) ist, der zumindest ein Stickstoffatom umfasst; und worin E, K und T, sofern vorhanden, N oder CH sind.

5. Verbindung nach einem der Ansprüche 1 bis 4 mit der Struktur:

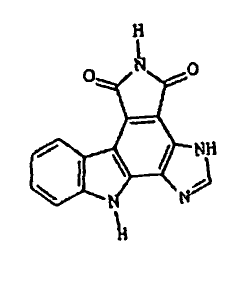

6. Verbindung nach einem der Ansprüche 1 bis 4 mit der Struktur:

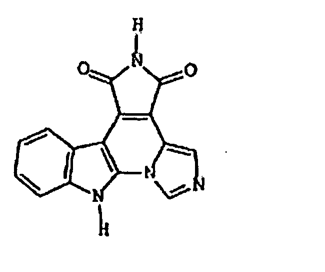

7. Verbindung nach einem der Ansprüche 1 bis 4 mit der Struktur:

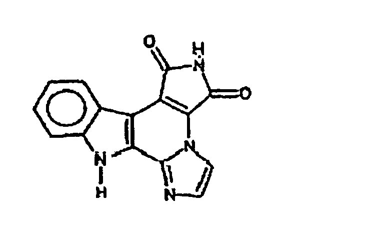

8. Verbindung nach einem der Ansprüche 1 bis 4 mit der Struktur:

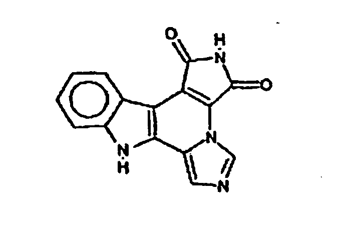

9. Verbindung nach einem der Ansprüche 1 bis 4 mit der Struktur:

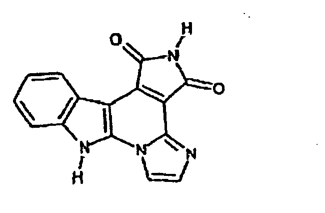

10. Pharmazeutische Formulierung, die eine Verbindung nach einem der Ansprüche 1 bis 9 und einen pharmazeutisch annehmbaren Träger umfasst.

11. Verbindung nach einem der Ansprüche 1 bis 9 zur Verwendung in einem Verfahren zur Behandlung eines Menschen- oder Tierkörpers.

12. Verwendung einer Verbindung nach einem der Ansprüche 1 bis 9 bei der Herstellung eines Medikaments zur Behandlung von Krebs.

13. Verwendung nach Anspruch 12, worin das Medikament Krebszellen für die Wirkungen von DNA-schädigenden Wirkstoffen auf Krebszellen empfindlich macht.

## Revendications

1. Composé ayant la structure: 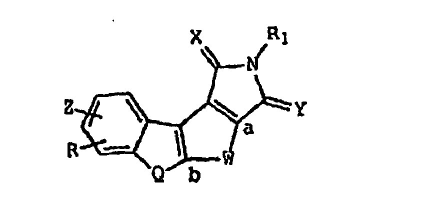 où:
W est sélectionné dans le groupe consistant en formule (i) ou (ii), où les structures sont comme suit:
(i) 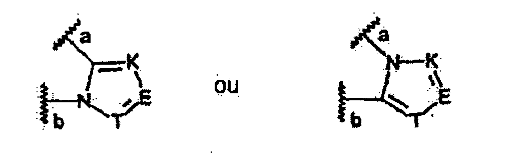 dans lesquelles K, E et T sont indépendamment sélectionnés dans le groupe consistant en: N, CR, et CZ, et où R et Z sont tels que définis ci-dessous; et
(ii) 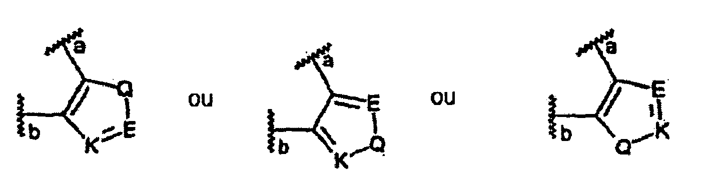
dans lesquelles K et E sont indépendamment sélectionnés dans le groupe consistant en: N, CR et CZ et où R, Z et Q sont tels que définis ci-dessous;
R₁ est sélectionné dans le groupe consistant en: R; RCO-; Ar₂CO-; et, Ar₂CH₂-, où Ar₂ est un substituant aromatique sélectionné dans le groupe consistant en: phényle, naphtyle, anthracyle, phénanthryle, furanne, pyrrole, thiophène, benzofuranne, benzothiophène, quinoléine, isoquinoléine, imidazole, thiazole, oxazole, et pyridine, et Ar₂ peut être facultativement substitué par R ou Z, où R et Z sont tels que définis ci-dessous;
R est sélectionné dans le groupe consistant en H; et un fragment de structure ayant un squelette linéaire, ramifié ou cyclique saturé ou insaturé contenant un à dix atomes de carbone où les atomes de carbone peuvent être facultativement substitués par un substituant sélectionné dans le groupe consistant en: -OH; -OR₃; -O₂CR₃, -SH; -SR₃; -SOCR₃; -NH₂; -NHR₃; -NH(R₃)₂; -NHCOR₃; NRCOR₃; -I; -Br; -Cl; -F; -CN; -CO₂H; -CO₂R₃; -CHO; -COR₃; -CONH₂; -CONHR₃; -CON(R₃)₂; -COSH; -COSR₃; NO₂; -SO₃H; -SOR₃; et -SO₂R₃, où R₃ est un groupe alkyle linéaire, ramifié ou cyclique, saturé ou insaturé, de un à dix carbones;
Z est un substituant facultatif sélectionné dans le groupe consistant en: H; -OH; -OR; -O₂CR; -SH; -SR; -SOCR; -NH₂; -NHR; -NH(R)₂; -NHCOR; NRCOR; -I; -Br; -Cl; -F; -CN; -CO₂H; -CO₂R; -CHO; -COR; -CONH₂; -CONHR; CON(R)₂; -COSH; COSR; -NO₂; -SO₃H; -SOR; et -SO₂R;
Q est NH; et
X et Y sont indépendamment sélectionnés dans le groupe consistant en: O; H; OH; et H₂.

2. Composé de la revendication 1, où R₁ est H ou CH₃.

3. Composé de l'une de la revendication 1 ou de la revendication 2, où X et Y sont oxygène.

4. Composé de l'une quelconque des revendications 1 à 3, où W est un cycle à cinq membres de formule (i) ou (ii) comprenant au moins un atome d'azote; et où E, K et T, quand ils sont présents sont N ou CH.

5. Composé de l'une quelconque des revendications 1 à 4, ayant la structure:

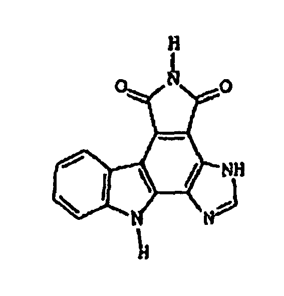

6. Composé de l'une quelconque des revendications 1 à 4 ayant la structure:

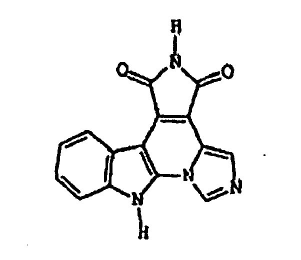

7. Composé de l'une quelconque des revendications 1 à 4, ayant la structure:

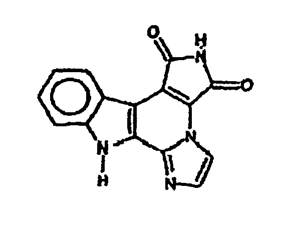

8. Composé de l'une quelconque des revendications 1 à 4, ayant la structure:

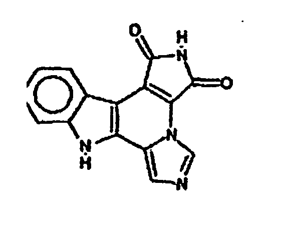

9. Composé de l'une quelconque des revendications 1 à 4, ayant la structure:

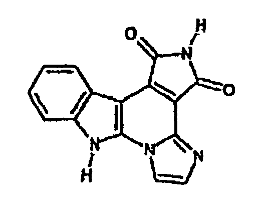

10. Formulation pharmaceutique comprenant un composé de l'une quelconque des revendications 1 à 9, et un support pharmaceutiquement acceptable.

11. Composé de l'une quelconque des revendications 1 à 9 à utiliser dans une méthode de traitement du corps humain ou animal.

12. Utilisation d'un composé de l'une quelconque des revendications 1 à 9 dans la fabrication d'un médicament pour le traitement du cancer.

13. Utilisation de la revendication 12, où le médicament sensibilise les cellules cancéreuses vis-à-vis des effets des agents dégradant l'ADN sur les cellules cancéreuses.
